# EUROPEAN PATENT APPLICATION

(11) **EP 3 028 721 A1**
(43) Date of publication of application: **08.06.2016**
(21) Application number: 14196674.7
(22) Date of filing: 05.12.2014
(51) Int. Cl.: A61K 49/00, A61K 41/00, A61K 31/00

(54) **Nanoparticle formulation having reverse-thermal gelation properties for injection**

(71) Applicant: Exchange Imaging Technologies GmbH, 64287 Darmstadt (DE)
(72) Inventor: GREB, Wolfgang, 40489 Düsseldorf (DE); BELEKE, Jean Philip, 51375 Leverkusen (DE)
(74) Representative: Arth, Hans-Lothar

(57) **Abstract**

The present invention refers to a pharmaceutical formulation for injection comprising nanoparticles as in vivo diagnostics and therapeutics. The present invention relates to an injectable pharmaceutical formulation for human medicine and/or veterinary use, comprising 5% to 50% per weight of a poloxamer, 0.1 nM to 10.0 µM nanoparticles and water or an aqueous buffer, wherein the pharmaceutical formulation is liquid at 4°C - 30°C and forms a gel at about 37°C, their use as an *in vivo* marker and methods of their preparation. The inventive formulation is useful for local control and prevention of spreading/diffusion of nanoparticles, and thus allows full utilization of their quantum physics properties for example as a tool to enable surgical precision of tumor removal; even without tumor specific epitope binding antibodies.

## Description

The present invention refers to a pharmaceutical formulation, preferably for injection and for human medicine and/or veterinary use, comprising 5% to 50% per weight of a poloxamer, 0.1 nM to 10.0 µM small nanoparticles and water or an aqueous buffer, wherein the formulation is liquid at 4°C - 30°C and forms a gel at 37°C. Furthermore, the present invention refers to the *in vivo* medical use of the inventive formulation and methods of their preparation.

### Background of the invention

Nanoparticles (NPs) have various potential uses in medicine such as diagnostic imaging or labelling, drug targeting, and therapeutic applications like tumor destruction by electronic field heating using magnetic iron nanoparticles. Nanoparticles are favourably used because of their small size but their high diffusion in biological tissues, especially that of small nanoparticles sized 1-20 nm, can be unwanted and present a problem when local retention and concentration maintenance at the site of local application is needed. Typically, after bodily applications of small NPs they quickly and randomly diffuse in and out of tissues and vessels and distribute into various organs. This poses the general problem of controlling their location and effects in respect to their desired use, local dosing and effective concentration, undesired effects, lack of effectiveness, uncontrollable distribution and remote accumulation.

When nanoparticles are applied to the body in solution and/or classical pharmaceutical formulations they typically diffuse very quickly due to themselves being much smaller than biological structures and the surrounding anatomy: they easily diffuse through tissue and cellular barriers, exit from blood vessels, enter cells, show unwanted accumulation in certain organs or are eliminated by active processes like macrophage capture.

The present invention relates to a method to reduce diffusion and keep NPs at the site of application, especially of manually targeted applications (such as injections, topical applications, etc). Therefore a formulation showing in situ gel formation due to temperature change has been developed. Such formulation allows liquid application and injection at room temperature and, through local gel formation at body respective tissue temperature at 37°C, a local retention for a determined and required period of time. This then effectively allows use of nanoparticle in a desired concentrated manner utilizing the typical nanoparticle effects (such as quantum physics and quantum chemistry effects, like strong fluorescence) of concentrated groups of NPs, which differentiates nanoparticles from larger particles.

The present invention allows usage of nanoparticles as toll for surgical precision of tumor removal even without tumor specific epitope binding antibodies. The inventive formulation is particularly designed for *in vivo* use in human medicine. Keeping NPs at a given site by a special formulation that promotes retention and higher local NP concentration is highly desirable for certain applications, prevents NP-aggregation (NP-aggregation negatively affects fluorescence), and stabilizes the wanted quantum effect and medical utility. Particularly body movements during diagnostic or therapeutic procedures like movements of muscle (via breathing or walking or change of body position for imaging) and or gastrointestinal peristalsis facilitate diffusion. Therefore a formulation containing nanoparticles, and especially small quantum dots, showing less diffusion but being easily injectable is particularly useful under these conditions requiring local application stability of the nanoparticles.

In the following a typical example for the usage of such a formulation is described:
When endoscopic removal of early found or pre-malignant colon cancer lesions is not possible, the removal of these lesions and in particular all malignant appearing lesions is the domain of minimally invasive surgery. However, the intraoperative location and identification of lesions previously detected by gut endoscopy is often difficult, particularly during laparoscopic surgery, where the surgeon has no other chance than visual inspection. Therefore, in recent years, numerous attempts have been made to establish methods to highlight areas to be resected by endoscopic or laparoscopic markers prior to surgery, mainly during pre-operative endoscopy. An ideal marker would be visible from both sides of the gut wall (transluminal marker).

The lack of accurate lesion localization and identification during laparoscopy may lead to start open surgery during the session (called conversion) with substantial time delay or resection of the wrong segment of bowel. Endoscopic tattooing is one of useful tool for the localization of small colorectal lesions especially in the laparoscopic setting. Thereby a sterile, non-pyrogenic ink (suspensions of carbon particles) is used as an endoscopic marker for marking polyps and lesions in the gastrointestinal tract. Other dyes that have been assessed as endoscopic markers include methylene blue, indigo carmine, hematoxylin, eosin, and indocyanine green (ICG). However, some studies have revealed complications resulting from this procedure, such as a very rapid diffusion into the surrounding tissue, dye bleaching, or a rapid fading. Furthermore in Europe, the use of ink is limited by the lack of approval for medical purposes. There is only one product being CE-certificated, namely Spot™ by GI Supply. Alternative localization techniques have included metal clips and barium enema.

Fluorescent Nanoparticles such as dye doped silica-or calcium phosphate-particles or surface modified semiconductor particles like those made of binary compounds such as lead sulfide, lead selenide, cadmium selenide, cadmium sulfide or cadmium telluride or from ternary compounds such as cadmium selenide sulfide are used as imaging agents, location markers or fiducials (X-ray detection of NPs in human). For instance, such Qdots^{®} or quantum dots are used because their fluorescence in terms of intensit and photostability exceeds the one of most other known fluorescence dyes. A direct, predictable relationship exists between the physical size of the quantum dots and the energy of the exciton and therefore, the wavelength and color of the emitted light. The small diameter between 2 and 20 nm allows using of quantum dots for different diagnostic purposes in *in vitro* studies. Nanoparticles and especially quantum dots coupled to proteins, oligonucleotides, small molecules, etc., may be used to direct binding of the nanoparticles to targets of interest.

Recently many new light emitting nanoparticles have been described, not only semiconductor particles but also dye doped particles. For example, WO 2010/030119 A2 and WO 2011/109214 A2 disclose fluorescent nanoparticles based on silica and Calcium phosphate. Nevertheless, the intensity and stability of their fluorescence is inferior compared to particles containing metal ions such as cadmium or zinc as semiconductor material. Cadmium free Qdots may contain gallium or indium as semiconductor material.

In general, the toxic potential, rapid diffusion and or organic distribution pattern, elimination/excretion kinetics of these nanoparticles as well as the amounts to be used has significantly limited their medical use in humans and especially their use for endoscopic marking of lesions. One of the main disadvantages of dyes used *in vivo* and especially of nanoparticles, due to their small size, is a rapid diffusion into the surrounding tissue or their uptake into and subsequently distribution by the blood system. Thus, *in vivo* there is only limited time period after staining e.g. for surgical removal once a tissue area has been marked. Diminished fluorescence due to diffusion requires injecting a higher dose, which is undesired in man because of toxic potential.

It would, therefore, be advantageous to find a pharmaceutical formulation containing nanoparticles, and particularly fluorescent nanoparticles, and more particularly quantum dots, showing less diffusion but being easily applicable, particularly easily injectable. Such a formulation would provide a prolonged staining of the local site and control of the injection and/or reduce the systemic exposure to possibly hazardous materials in living animal or man, such as cadmium containing quantum dots. Ideally, such foreign and toxic marker is to be removed and cut out by the surgeon with the tissue to be excised, at least when the marker does not consist of naturally occurring ingredients.

In another example for therapeutic local tissue targeting, ferrofluids (colloidal liquids made of super paramagnetic nanoparticles) are used for drug targeting and held at the bodily target site by positioned magnets to keep the concentration high at this - typically a tumor - position. In such "magnetic field" treatment the iron nanoparticles are heated by externally applied electric fields, with the consequence of local cell destruction. Magnetic nanoparticles are further suitable for MRI (magnetic resonance image) diagnostics. Application of such iron oxide nanoparticles is also improved by using an inventive formulation containing such super paramagnetic nanoparticles.

Examples for suitable super paramagnetic nanoparticles are γ-Fe₂O₃, Fe₃O₄, MnFe₂O₄, NiFe₂O₄, CoFe₂O₄, and any mixtures thereof. The two main forms are magnetite (Fe₃O₄) and its oxidized form maghemite (γ-Fe₂O₃). For example, WO 01/74337 A1 and EP 1378239 A1 disclose super paramagnetic nanoparticles which may be part of a formulation according to the invention.

Furthermore, photothermal therapy (PTT) is extensively investigated as an alternative procedure to treat cancer. In PTT, heat is generated within the targeted carcinoma tissue through absorption of applied laser light, causing thermal injury and cell death. Thereby the light sensitivity of targeted tissue is increased by use of dye (particularly chromophores absorbing light in the near infrared (NIR) region of the electromagnetic spectrum) doped nanoparticles such as gold nanoparticles, gold nanorods, gold nanoshells (silica particles coated with a nanometer-thick gold shell), nanoparticles containing organic chromophores like indocyanine green (ICG), and silver/organic dye composites nanoparticles. For both tumor-imaging and photodynamic anticancer therapy applications, the delivery of chromophore (such as ICG) containing nanopartiocles to the tumor site and accumulation and retention in this site are the crucial steps.

It is the objective of the present invention to provide a pharmaceutical formulation which allows convenient/easy fluid injection of nanoparticles but having less diffusion resulting in a prolonged and controlled property, i.e. fluorescence, of the nanoparticles at the site of local deposition on, at and/or in the tissue of a living body after local application, implantation and or injection.

The objective of the present invention is solved by the teaching of the independent claims. Further advantageous features, aspects and details of the invention are evident from the dependent claims, the description, the figures, and the examples of the present application.

### Description of the invention

To avoid the rapid decay of fluorescence a novel pharmaceutical formulation was envisaged which should have liquid state at low temperatures (for storage and injection) while it should become a gel like status at body temperature to increase local retention time in the target tissue after application.

The inventors could surprisingly show that nanoparticles, such as quantum dots, embedded in a pharmaceutical formulation containing poloxamers and an aqueous excipient remain longer at the site of injection, while unformulated nanoparticles diffuse into the surrounding tissue and are rapidly taken up by the blood and lymphatic system. The term "unformulated nanoparticles" refers to nanoparticles which are dissolved or suspended in water or an aqueous buffer without further ingredients and especially without glycol oligomers or glycol polymers. The term "glycol oligomers or glycol polymers" refers to oligomers or polymers containing glycol units -O-CH₂-CH₂-O-.

Therefore, the present invention relates to a pharmaceutical formulation for injection comprising 5% to 50%, preferred 10% to 40% per weight of at least one poloxamer, 0.1 nM to 10.0 µM of at least one nanoparticle, preferably 1.0 nM to 6.0 µM, more preferably 5.0 nM to 4.0 µM of at least one nanoparticle and water or an aqueous buffer, wherein the pharmaceutical formulation is liquid at 4°C - 30°C, preferably at 4°C - 25°C, more preferably at 4°C - 20°C and forms a gel at about 32°C to 37°C (being semisolid). The nanoparticles are preferably ferromagnetic or fluorescent. The pharmaceutical formulations disclosed herein are intended for human medicine and/or veterinary use. In between the temperature range of about 25 - 30°C to about 37°C the pharmaceutical formulation is in the intermediate state of becoming more and more gel like with increasing temperature and less fluid.

Another embodiment of the present invention relates to a pharmaceutical formulation for injection comprising 17% to 20% per weight of poloxamer 407, preferably 18% per weight of poloxamer 407, and 3% - 15% per weight poloxamer 188, preferably 5% - 10% per weight poloxamer 188, 0.1 nM to 10.0 µM of at least one nanoparticle, preferably 1.0 nM to 6.0 µM, more preferably 5.0 nM to 4.0 µM of nanoparticles and water or an aqueous buffer.

Preferably the present invention relates to a pharmaceutical formulation consisting of 0.1 to 14.0% per weight quantum dots, preferably of 1.0 to 10.0% per weight quantum dots, more preferably of 2.0 to 5% per weight quantum dots. A particularly preferred formulation consists of 15% per weight poloxamer 407, 15% per weight poloxamer 188, 2.0 to 3.5% per weight quantum dots and 66.5 to 68% per weight Aqua ad injectabilia. Another preferred embodiment of the present invention relates to a pharmaceutical formulation consisting of 7% per weight quantum dots, 18% per weight poloxamer 407, 5% per weight poloxamer 188 and 70% per weight Aqua ad injectabilia. Preferred is further a pharmaceutical formulation consisting of 7% per weight quantum dots, 18% per weight poloxamer 407, 10% per weight poloxamer 188 and 65% per weight Aqua ad injectabilia.

A further preferred embodiment of the present invention relates to a formulation consisting of 2% per weight quantum dots, 18% per weight poloxamer 407, 5% per weight poloxamer 188 and 75% per weight Aqua ad injectabilia. Preferred is further a formulation consisting of 3,5 % per weight quantum dots, 18% per weight poloxamer 407, 10% per weight poloxamer 188 and 68.5% per weight Aqua ad injectabilia.

The term "nanoparticle" or "ultrafine particles" as used herein refers to a small object that behaves as a whole unit with respect to its transport and properties and have a diameter between 1 and 500 nanometers in size, preferably between 1 and 100 nanometers. Individual molecules are not referred to as nanoparticles.

The term "poloxamer" as used herein refers to poly(ethylene glycol)-*block-*poly(propylene glycol)-*block*-poly(ethylene glycol), block copolymers of ethylene oxide and propylene oxide falling under the following general formula:

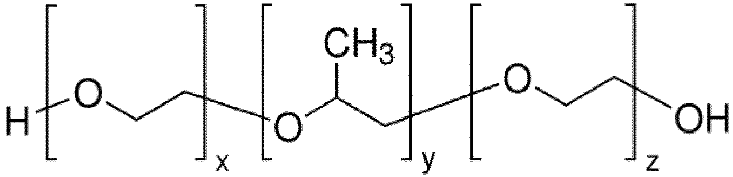

Poloxamers are also known by the trade names Synperonics^{®}, Pluronics^{®}, Lutrol^{®} and Kolliphor^{®}. They are widely used in pharmaceuticals, cosmetics and nutraceuticals and recognized as very safe excipients. Because the lengths of the polymer blocks can be customized, many different poloxamers exist having slightly different properties. The generic term "poloxamer" is commonly followed by three digits, the first two digits x 100 give the approximate molecular mass of the polyoxypropylene core, and the last digit x 10 gives the percentage polyoxyethylene content (e.g., Poloxamer 407 or P407 = Poloxamer with a polyoxypropylene molecular mass of 4,000 g/mol and a 70% polyoxyethylene content). It is preferred that the poloxamer contained in the pharmaceutical formulation according to the present invention is poloxamer 407. Even more preferred is that poloxamer 407 is used in combination with poloxamer 188. In regard to Poloxamer 407, x and z in the general formula have the value of 101 and y is 56, wherein for poloxamer 188 x and z have the value of 80 and y is 27.

Preferably the present invention relates to a pharmaceutical formulation, wherein the nanoparticles are selected from the group comprising or consisting of: semiconductor nanoparticles, quantum dots (of different metal content), dye-doped silica nanoparticles (coupled with or without targeting ligands), preferably fluorescent dye-doped silica nanoparticles, dye dope calcium particles (coupled with or without targeting ligands), dye labeled liposomes and fluorescent carbon nanoparticles (coupled with targeting ligands or without targeting ligands), iron oxide nanoparticles eg called SPION), gold nanoparticles, gold nanorods, gold nanoshells (silica particles coated with a nanometer-thick gold shell), nanoparticles containing organic chromophores like indocyanine green (ICG), and silver/organic dye composites nanoparticles, nanoparticles containing a gadolinium oxide core and combinations of the afore-mentioned nanoparticles.

Consequently, a particularly preferred pharmaceutical formulation according to the invention contains ferromagnetic nanoparticles or a combination of ferromagnetic nanoparticles and fluorescent nanoparticles. These formulations are especially suitable for treatments, which include heating up a tumor site after injection by external fields and thus destroy local tumor tissue.

Suitable dye-doped silica nanoparticles and in particular fluorescent dye-doped silica nanoparticles and methods for their preparation are for example disclosed in WO2011/109214 A2 and in WO2010/030119 A2. Dye labeled, fluorescent liposomes can be purchased, for example from FormuMax Scientific, USA. Fluorescent carbon nanoparticles which can be formulated as described herein, and their synthesis has been described by Susanta Kumar Bhunia et al. (Carbon Nanoparticle-based Fluorescent Bioimaging Probes; Sci Rep. 2013; 3: 1473).

It is particularly preferred that fluorescent nanoparticles of the pharmaceutical formulation according to the invention are quantum dots. The term "quantum dots" as used herein refers to nanocrystals made of semiconductor materials with diameters in the range of 2-10 nanometers. It is important that the diameters are small enough to exhibit quantum mechanical properties. The term nanocrystals refers to nanometer-sized single crystals, or single-domain ultrafine particles. Preferred quantum dots are nanometer-scale (roughly protein-sized) atom clusters, containing from a few hundred to a few thousand atoms of a semiconductor material (such as cadmium mixed with selenium or tellurium), which has been coated with an additional semiconductor shell (such as zinc sulfide) to improve the optical properties of the material.

Therefore, one preferred embodiment of the present invention relates to pharmaceutical formulations, wherein the quantum dots contain a core made of cadmium mixed with selenium or tellurium and are coated with zinc sulfide. Other suitable quantum dots may be selected from the group comprising or consisting of: InP/ZnS core/shell quantum dots, Mn/ZnSe quantum dots, Mn doped ZnO quantum dots, CuInS/ZnS core/shell quantum dots, Gold NanoRods, ZnCuInS/ZnS core/shell quantum dots, ZnSe/ZnS CuInS/ZnS core/shell quantum dots, CuInS/ZnS core/shell quantum dots and InP/ZnS quantum dots. In general, many different kinds of quantum dots can be purchased, for example from life technolgies, mkNANO or Mesolight. The quantum dots are used in the pharmaceutical formulation in an amount of 0.1 nM to 10.0 µM of at least one quantum dot, preferably 1.0 nM to 6.0 µM, more preferably 5.0 nM to 4.0 µM of at least one quantum dot. The term "at least one quantum dot" refers to at least one sort of quantum dots and thus comprises also mixtures of different sorts of quantum dots.

Preferred as fluorescent nanoparticles are nanocrystals containing a core made of atoms of a semiconductor material, which has been coated with an additional semiconductor shell. Preferred are further pharmaceutical formulations according to the invention, wherein the nanocrystals contain a core of cadmium selenide or telluride and are coated with zinc sulfide.

The present invention relates also to pharmaceutical formulations containing nanoparticles which are linked to biomolecules. Thus, one aspect of the present invention is directed to a nanoparticle (being e.g. fluorescent or ferromagnetic) containing a pharmaceutically active agent. These biomolecules or biogenic substances or pharmaceutically active agents include large macromolecules such as proteins, polysaccharides, lipids, antibodies, and nucleic acids, as well as small molecules such as primary metabolites, secondary metabolites, peptides, small molecule drugs and natural products. These bioconjugates (nanoparticle linked or conjugated to biomolecules) or targeted nanoparticles are suitable for specific or controlled staining of a cell type (such as cancer cells) or a specific tissue or substructures (specific molecules in the cell membrane or specific structures in a tissue). The present invention relates further to pharmaceutical formulations, wherein the nanoparticles contain a ligand selected from the group consisting of peptides, antibodies, and oligonucleotides. Suitable target molecules or ligands which can be coupled and preferably covalently coupled to the nanoparticles are biologically active structures, e.g. protein receptors, ligands, enzymes, substrates, antibodies, antigens, chelators and the like. The ligands of the targeted nanoparticles can for example specifically interact with tumor epitopes and would therefore allow marking tumors to assess the spread of tumors or their metastases and single cell flotation.

The present invention relates also to pharmaceutical formulations containing nanoparticles which are linked to polyethylenglycol (PEGylated nanoparticles). Another embodiment of the present invention relates to a pharmaceutical formulation comprising nanoparticles, especially fluorescent nanoparticles, containing a pharmaceutically active agent such as a drug for use in medicine. These pharmaceutical formulations could combine diagnosis or monitoring with therapy. The fluorescent nanoparticles allow tracking of the active agent after administration of the pharmaceutical formulation.

Another embodiment of the present invention relates to a pharmaceutical formulation according to the invention, wherein the solvent is water. Preferred aqueous buffer are phosphate buffer (PP) or phosphate buffered saline. Phosphate buffers are based on salts of phosphoric acid. Phosphoric acid has multiple dissociation constants, therefore, one can prepare phosphate buffers with varies pH values. The buffer is most commonly prepared using dihydrogen phosphate and dibasic monohydrogen phosphate. One can for example use monosodium phosphate and its conjugate base, disodium phosphate. Also potassium phosphates are commonly used. Phosphate buffered saline (PBS) is a water-based salt solution containing sodium phosphate, and potassium chloride and potassium phosphate. PBS is often used in biology and pharmaceutical formulations because it is isotonic and non-toxic to cells.

The pharmaceutical formulation according to the present invention can effectively be used for optimal endoscopic or coloscopic application and laparoscopic visualization, as well as injected into tissue from external surfaces or deep injection, because it has the following characteristics:
- Liquid at temperatures from 4°C to room temperature.
- Liquid during endoscopic procedure and filling of the injection device.
- Injectable by an injection device, i.e endoscopy capillary
- Becoming semisolid or gel-like at body temperature (within 30 minutes)
- Adequate stability and mechanical properties after injection in the tissue
- stable at pH of 7.0 to 8.6 (particularly at physiological pH values)
- Excipients of pharmaceutical grade quality, approved for injections in humans
- Excipients of non-animal origin

Therefore, another embodiment of the present invention relates to the pharmaceutical formulations according to the present invention for use preoperatively, in particular in the marking or staining of specific areas in the patient's body. The pharmaceutical formulations according to the present invention are particularly suitable for use as a marker of bodily areas applied by injection directly into an organ or diseased body region and further for use as a marker of tumor areas to enable surgical precision of tumor removal.

The pharmaceutical formulations according to the present invention are especially developed for use in preoperative staging of bodily areas and especially gastrointestinal areas applied by injection during an endoscopic procedure. The pharmaceutical formulations disclosed herein are used for different type of inspections into body spaces like bronchoscopy, otolayngoscopy, cystoscopy, colposcopy, etc. as well as in open surgery and marking of specific locations for the purpose of visually guided surgery, as well as fiducial markers for computer surgery. The newest surgery machines require such markers for orientation purposes especially when the patient moves during surgery.

Besides the effect that the pharmaceutical formulations according to the present invention remain at the site of injection for a prolonged time (typically some days). This enables to mark the lesion in the intestine or colon longer and makes it easier to find this lesion until and during surgery. In addition when using nanoparticles containing heavy metal or toxic nanoparticles it is preferred that as many nanoparticles as possible remain at the injection site /marked area because with tumor excision the marked area (lesion; tumor tissue) and also the nanoparticles are removed from the patient's body and none or less unwanted systemic exposure will take place.

The use of poloxamers in the inventive formulation have advantages that they favourably bind to cell membranes, as has been shown with the inventive formulation in binding experiments at human tumor tissue slices. Nanoparticles without special binding groups, like Quantum dots do not themselves bind to cell membranes but the poloxamers may mediate binding to cell membranes.

Alternatively, the pharmaceutical formulations according to the present invention are suitable for use as a marker of areas in the skin, preferably in the dermis of skin. The pharmaceutical formulation according to the present invention containing super paramagnetic nanoparticles is suitable for use in photothermal therapy.

The inventors found that it is preferred that in the pharmaceutical formulation according to the invention, the nanoparticles are not integrated in the self-forming micelles, which are generated by the poloxamers after dissolution in water. Such poloxamer formulations have been previously described for many water insoluble compounds. However, the pharmaceutical formulation of the present invention contains nanoparticles being dispersed between the micelles formed by poloxamers. This means in case the inventive pharmaceutical formulation is liquid both the poloxamer micelles and the nanoparticles are dispersed in the water or the aqueous buffer and in case of semi-solid or gel-like state the nanoparticles are dispersed in the gel matrix build by the poloxamers. It is especially preferred if the nanoparticles used within the inventive pharmaceutical formulation are hydrophilic.

Further embodiment of the present invention relates to a method for the preparation of the pharmaceutical formulation according to the present invention comprising or consisting of the following steps:
a) dissolving at least one poloxamer in water or in an aqueous buffer;
b) stirring for at least 20 hours at 4°C; and
c) adding at least one nanoparticle.

The term "at least one poloxamer" obviously refers to at least one sort of poloxamer and not a single poloxamer molecule. Analogous the term "at least one nanoparticle" refers to at least one kind of nanoparticles (preferred are fluorescent and/or paramagnetic nanoparticles) and not a single nanoparticle molecule.

A preferred embodiment of the present invention relates to a method for the preparation of a pharmaceutical formulation comprising or consisting of the following steps:
a) dissolving at least one poloxamer in water being precooled to 4°C - 8°C or in an aqueous buffer being precooled to 4°C - 8°C;
b) stirring for at least 20 hours at 4°C; and
c) adding at least one nanoparticle.

Another preferred embodiment of the present invention relates to a method for the preparation of a pharmaceutical formulation comprising or consisting of the following steps:
a) heating up at least one poloxamer to a temperature between 70°C - 80°C;
b) dissolving the melted poloxamer in water heated up to a temperature between 70°C - 80°C or in an aqueous buffer heated up to a temperature between 70°C - 80°C;
c) cooling down the obtained mixture to 4°C while stirring the obtained mixture;
d) stirring for at least 20 hours at 4°C; and
e) adding at least one fluorescent nanoparticle.

This method involves the mixing under stirring of an aqueous buffer or water which is heated to a temperature within the temperature range of 70°C to 80°C with at least one (sort of) poloxamer which is also heated to a temperature within the temperature range of 70°C to 80°C. Thereafter the obtained mixture is preferably allowed to cool to room temperature under stirring. When room temperature is reached, active cooing, for instance by a cooling bath with ice water, is applied and the obtained mixture is cooled to 4°C and stirred at 4°C for at least 20 hours.

Another aspect the present invention refers to pharmaceutical formulations obtainable according to one of the above described methods. These methods ensure that the at least one poloxamer generates micelles and that the added fluorescent nanoparticles are not included into these micelles but are finely dispersed in the pharmaceutical formulation (between the micelles formed by poloxamers) or respectively in the gel-matrix at higher temperatures.

The pharmaceutical formulation for injection or pharmaceutical composition for injection according to the present invention comprising 5% to 50% per weight of a poloxamer, 0.1 nM to 10.0 µM of at least one nanoparticle, preferably 1.0 nM to 6.0 µM, more preferably 5.0 nM to 4.0 µM of at least one fluorescent nanoparticle and water or an aqueous buffer and in particular the pharmaceutical formulation for injection or pharmaceutical composition for injection according to the present invention comprising 17% to 20% per weight of poloxamer 407, preferably 18% per weight of poloxamer 407, and 3% - 15% per weight poloxamer 188, preferably 5% - 10% per weight poloxamer 188, 0.1 nM to 10.0 µM of fluorescent nanoparticles, preferably 1.0 nM to 6.0 µM, more preferably 5.0 nM to 4.0 µM of fluorescent nanoparticles and water or an aqueous buffer will typically be administered by injection. The pharmaceutical formulations may be administered together with further excipients selected with respect to the intended form of administration and consistent with conventional pharmaceutical practices.

Where necessary, the pharmaceutical formulation may also include a solubilizing agent and a local anesthetic to ease pain at the site of the injection. For liquid compositions, especially in the form of solutions for parenteral injections, the pharmaceutical formulation of the present invention may be combined with additives for preservation, for adjusting pH or osmolarity, and/or for providing stability or long term stability. Nevertheless, the inventors could show that this additive diminishes fluorescence of the pharmaceutical formulation, thus, it is preferred that the pharmaceutical formulation according to the invention does not contain D,L alpha-tocopherol.

Pharmaceutical compositions for injection are typically sterile solutions. Therefore it is preferred that the aqueous portion of the pharmaceutical formulation is endotoxin free water such as highly purified water or water for the purpose of injection (Aqua ad injectabilia) or is based on highly purified water or water for the purpose of injection (Aqua ad injectabilia). Furthermore the pharmaceutical formulation may be sterilized using commonly known methods, such as methods including ionizing radiation (gamma and electron-beam radiation), and gas (ethylene oxide, formaldehyde).

Different sugars such as saccharose or trehalose may be added as cryoprotecting agents or for adjusting osmolarity of the solution. Polyalcohols, for example mannitol and sorbitol, may be used as stabilizer or for adjusting osmolarity.

Furthermore the composition may contain amino acids, such as glycine, arginine, leucine, carnosine or proline, as stabilizer. Further stabilizer of the solution may be urea or human serum albumin. In addition salts, such as NaCl, may be added in low concentration for stabilization of liquid form preparations.

Benzyl alcohol may be used as a bacteriostatic preservative at low concentration in the composition and especially in liquid form preparations of the present invention. In addition antioxidants, such as methionine, may be used.

### Description of the Figures

- Figure 1: shows a standard curve of fluorescence (A) in regard to different amounts of investigated, inventive pharmaceutical formulation determined by fluorimetric measurement and a standard curve for cadmium determination (B).
- Figure 2: shows quantum dot concentrations on the apical side and in the cellular layer after 1 h (A) and 3h (B) incubation. All quantum dot concentrations on basal sides were below quantification level. Therefore these values are not displayed.
- Figure 3: shows cadmium concentrations on the apical side and in the cellular layer after 1h (A) and 3h (B) incubation. Cadmium concentrations on basal sides were below quantification level. Therefore these values are not displayed.
- Figure 4: shows characteristics of inflammatory reactions of mild, moderate and severe intensity in the skin.
- Figure 5: shows shear-dependent viscosity behavior of Gel-F4 (A), Gel-F5 (B), QDot 655 F4 (3.5%; C) and QDot F5 (7%; D) (results of example 11).

The following examples are included to demonstrate preferred embodiments of the invention. It is to be understood that the forms of the invention shown and described herein are to be taken as examples of embodiments. However, those of skill in the art should, in light of the present disclosure, appreciate that many changes can be made in the specific embodiments which are described by the examples and still obtain a like or similar result without departing from the spirit and scope of the invention.

### EXAMPLES

### Example 1: Preparation of an inventive pharmaceutical formulation

150mg of poloxamer 407 (Lutrol^{®}F127 from BASF; Germany) and 150mg poloxamer 188 (Lutro^{®}F68 from BASF; Germany) were mixed together with 20mg DL-α-Tocopherol (Hänseler) and this mixture was added to 610.2mg pre-cooled water (Aqua ad injectabilia from BBraun Melsungen; approx. 5°C) in a transparent glass beaker and constant agitation with a magnetic stirring bar was kept for 24 - 48 hours at 4°C.

Subsequently, 69.8 mg of Qdot^{®}655 solution (Qdot^{®}655 dipeptide quantum dots *commercial use*; Lot Number: 812401- 1 and 812401- 2, in 50mM borate buffer, pH 8.3 from life technologies brand of Thermo Fisher Scientific, USA) was added to 930.2 mg of the pharmaceutical formulation and mixed to homogeneity at room temperature. Resulting pharmaceutical formulations were stored at 4°C (2°C to 8°C) under light protected conditions.

### Example 2: Preparation of an inventive pharmaceutical formulation

180mg of poloxamer 407 (Lutrol^{®}F127 from BASF; Germany) and 50mg poloxamer 188 (Lutrol^{®}F68 from BASF; Germany) was added to 700.2mg pre-cooled water (Aqua ad injectabilia from BBraun Melsungen; approx. 5°C) in a transparent glass beaker and constant agitation with a magnetic stirring bar was kept for 24 - 48 hours at 4°C.

Subsequently, 69.8 mg of Qdot^{®}655 solution (Qdot^{®}655 dipeptide quantum dots *commercial use*; Lot Number: 812401- 1 and 812401- 2, in 50mM borate buffer, pH 8.3 from life technologies) was added to 930.2 mg of the pharmaceutical formulation and mixed to homogeneity at room temperature. Resulting pharmaceutical formulations were stored at 4°C (2°C to 8°C) under light protected conditions.

### Example 3: Preparation of an inventive pharmaceutical formulation

180mg of poloxamer 407 (Lutrol^{®}F127 from BASF; Germany) and 100mg poloxamer 188 (Lutrol^{®}F68 from BASF; Germany) was added to 650.2mg pre-cooled water (Aqua ad injectabilia from BBraun Melsungen; approx. 5°C) in a transparent glass beaker and constant agitation with a magnetic stirring bar was kept for 24 - 48 hours at 4°C.

Subsequently, 69.8 mg of Qdot^{®}655 solution (Qdot^{®}655 dipeptide quantum dots *commercial use*; Lot Number: 812401- 1 and 812401- 2, in 50mM borate buffer, pH 8.3 from life technologies) was added to 930.2 mg of the pharmaceutical formulation and mixed to homogeneity at room temperature. Resulting pharmaceutical formulations were stored at 4°C (2°C to 8°C) under light protected conditions.

### Example 4: Preparation of an inventive formulation using "hot process"

180mg of poloxamer 407 (Lutrol^{®}F127 from BASF; Germany) and 100mg poloxamer 188 (Lutrol^{®}F68 from BASF; Germany) was weighted out in a glass tube and heated up to 70°C - 80°C. Aqua ad injectabilia (BBraun Melsungen) was heated up to 70°C - 80°C and mixed with the melted poloxamers. Constant agitation with a magnetic stirring bar was kept for 24 - 48 hours at 4°C.

Subsequently, 69.8 mg of Qdot^{®}655 solution (Qdot^{®}655 dipeptide quantum dots *commercial use*; Lot Number: 812401- 1 and 812401- 2, in 50mM borate buffer, pH 8.3 from life technologies) was added to 930.2 mg of the pharmaceutical formulation and mixed to homogeneity at room temperature. Resulting pharmaceutical formulations were stored at 4°C (2°C to 8°C) under light protected conditions.

### Example 5: Preparation of an inventive pharmaceutical formulation with PP

0,1M Phosphat buffer pH 7.4 were prepared: 6.805 g of potassium dihydrogen phosphate were dissolved in aqua ad injectabilia to obtain 500 ml of 0.1 M stock solution A. 8.90 g of disodium hydrogenphosphate dihydrate were dissolved at room temperature in aqua ad injectabilia to obtain 500 ml Stock solution B. 81.8 ml stock solution B and 18.2 ml stock solution A were mixed to give a final volume of 100 ml 0.1 M phosphate buffer, pH 7.4. After sterile filtration (0.22µm) buffer was stored at 4°C.

180mg of poloxamer 407 (Lutrol^{®}F127 from BASF; Germany) and 50mg poloxamer 188 (Lutrol^{®}F68 from BASF; Germany) was weighted out in a glass tube and heated up to 70°C - 80°C. Phosphate buffer (0,1 M, pH 7.4) was heated up to 70°C - 80°C and mixed with the melted poloxamers up to 930.2 mg. Constant agitation with a magnetic stirring bar was kept for 24 - 48 hours at 4°C.

Subsequently, 69.8 mg of Qdot^{®}655 solution (Qdot^{®}655 dipeptide quantum dots *commercial use*; Lot Number: 812401- 1 and 812401- 2, in 50mM borate buffer, pH 8.3 from life technologies) was added to 930.2 mg of the pharmaceutical formulation and mixed to homogeneity at room temperature. Resulting pharmaceutical formulations were stored at 4°C (2°C to 8°C) under light protected conditions.

### Example 6: Preparation of an inventive pharmaceutical formulation with PBS

180mg of poloxamer 407 (Lutrol^{®}F127 from BASF; Germany) and 50mg poloxamer 188 (Lutrol^{®}F68 from BASF; Germany) was weighted out in a glass tube and heated up to 70°C - 80°C. Phospahte bufferd saline (PBS; 12mM phosphate, 137mM NaCl and 2.70mM KCI, pH 7.4) was heated up to 70°C - 80°C and mixed with the melted poloxamers up to 930.2 mg. Constant agitation with a magnetic stirring bar was kept for 24 - 48 hours at 4°C.

Subsequently, 69.8 mg of Qdot^{®}655 solution (Qdot^{®}655 dipeptide quantum dots *commercial use*; Lot Number: 812401- 1 and 812401- 2, in 50mM borate buffer, pH 8.3 from life technologies) was added to 930.2 mg of the pharmaceutical formulation and mixed to homogeneity at room temperature. Resulting pharmaceutical formulations were stored at 4°C (2°C to 8°C) under light protected conditions.

### Example 7: Evaluation of physical formulation properties

### A: Appearance and pH value

The appearance of the pharmaceutical formulations was assessed by visual observation: homogeneity, phase separation. Furthermore, color was assessed in quantum dots containing formulations. pH values of the pharmaceutical formulations (after 1:10 w/v dilution with distilled water) was detected using a 766 Knick pH meter.

### B: Visual detection of formulation viscosity

A simple test tube inverting method was used to determine formulation viscosity. 5 ml of the respective pharmaceutical formulation consisting of different amounts of poloxamers (w/o additional excipients) were transferred to glass vials sealed with lamellar plugs and placed in a thermostat (4°C) water bath. Temperature was stepwise increased up to 37°C and left to equilibrate for 10 minutes at each new setting. At predefined temperatures viscosity was visually detected by turning the glass tubes upside down and classifying the flow properties of the transparent pharmaceutical formulation into four categories:
1: liquid like water
2: slightly viscous
3: viscous to highly viscous
4: semisolid

This method is easy to perform but shows limitations: Exact detection of gelation temperature is not possible as sol-gel transition occurs within a large temperature interval which is influenced by experimental set-up.

### C: Injectability of formulations at room temperature

Injectability of formulations was assessed by aspiration of 1 ml of the respective pharmaceutical formulation into a 1 ml syringe to which a 0.9 x 40 mm cannula was attached. The force necessary to fill the syringe through the cannula was assessed as
1: force comparable to aspiration of water
2: force comparable to aspiration of oil
3: strong forces required

### D: Measurement of gel strength

Gel strength of selected formulations was assessed by the following procedure:
1 ml g of the respective pharmaceutical formulation was poured into a 2 ml screw capped glass vial and gelation was induced by exposure of the pharmaceutical formulation to 37°C. Gel formation was observed within several minutes, usually within 1 to 10 minutes. To ensure full gel formation samples were incubated at 37°C for approx. 30 minutes.

After gel formation 1 ml of prewarmed water (37°C) was added, the vials screwed again and incubated in the water bath with gentle shaking at 37°C (30 rpm).

The gel strength was determined as the time necessary for dissolution of the gel.

### RESULTS

In a first series of experiments "empty" hydrogels i.e. hydrogels without addition of Qdots^{®}655 were tested with regard to their thermo sensitive behavior, transparency and pH value. Amount of Qdot^{®}655 was substituted by.

Empty gels in concentrations of 15%, 20%, 25% and 30% of Poloxamer 407 were prepared analogue to example 1.

Pharmaceutical formulations containing 15% of Poloxamer 407 appeared liquid or slightly viscous, respectively, at temperatures between 4°C and 22°C. Gel strength increased with temperature: at 30°C formulations appeared viscous and became semisolid at 37°C. Formulations consisting of 20% or 25% of Poloxamer 407 were liquid at 4°C and became semisolid at room temperature; a hydrogel prepared with 30% of Poloxamer 407 was already highly viscous at 4°C.

Poloxamer 407 formulations appeared transparent and colorless; they were homogeneous after 24 to 48 hours at temperatures of approx. 4°C.

### Effect of various Poloxamer 407 / 188 ratios:

Properties of pharmaceutical formulations containing Poloxamer 407 as main excipient, such as thermo sensitive behavior of pharmaceutical formulations, can be modified in the presence of different additives. Poloxamer 188 in different concentrations (5%, 10%, 15%, 20%) was added to Poloxamer 407 (15%, 20%, 25%, 30%) and the resulting pharmaceutical formulations assessed as to their physical properties. Addition of Poloxamer 188 led to change of gel formation:

Pharmaceutical formulations composed of 15% Poloxamer 407 and 5% to 15% of Poloxamer 188 appeared viscous at room temperature, but did not achieve the semisolid state at 37°C. This could be achieved only by addition of Poloxamer 188 at higher concentrations than 15%. Pharmaceutical formulations consisting of 20%, 25% or 30% of Poloxamer 407 were solid at 37°C; however, viscosity at lower temperatures was high as well.

Pharmaceutical formulations composed of 15% or 20% Poloxamer 407 showed the best physical properties according to thermo sensitive behaviour. Thus, gels were tested using 18% of Poloxamer 407 in combination with different concentrations of Poloxamer 188 (5%, 10%, 15%, 18%. Pharmaceutical formulations with 18% Poloxamer 407 and either 5% or 10 % of Poloxamer 188 were liquid at 4°C, slightly viscous at 22°C and semisolid at 37°C. These compositions showed the required thermo sensitive characteristics. Viscosity at 4°C and room temperature increased in presence of higher Poloxamer 188 concentrations.

### Influence of phosphate buffers on Poloxamer 407 / 188 preparations

The influence of buffers on Poloxamer 407 / 188 formulations was tested by preparing different pharmaceutical formulations with either phosphate buffer (PP, 0.1 M, pH 7.4) or phosphate buffered saline (PBS, 0.1 M, pH 7.4) as aqueous phase.

Addition of buffers instead of pure water did not change the viscous properties of the pharmaceutical formulations.

Influence of DL-α-Tocopherol on Poloxamer 407 / 188 preparations DL-α-Tocopherol is known to reduce the critical micelle concentration and is expected to stabilize pharmaceutical formulations according to the invention. DL-α-Tocopherol was added to melted Poloxamer 407 / 188 mass previous to the addition of water.

Poloxamer 407 / 188 formulations were prepared with DL-α-Tocopherol in different concentrations: 0.5%, 1.0%, 1.5% or 2.0% according to example 1. Pharmaceutical formulations containing DL-α-Tocopherol appeared transparent and slightly yellowish in dependence of the tocopherol concentration. DL-α-Tocopherol in pharmaceutical formulations composed of 15% Poloxamer 407 and 5% or 10% Poloxamer 188 appeared transparent and homogeneous. The fluorescence properties are reduced as compared to identical pharmaceutical formulations without D,L-α-Tocopherol.

### Nanoparticle containing pharmaceutical formulations

After selecting most promising "empty" formulations (composed of 15% Poloxamer 407 and 5% or 10% Poloxamer 188) based on their thermo sensitive properties, the same preparations were loaded with Qdot^{®}655. Qdots appeared evenly spread; no turbidity or sedimentation could be observed even after two months of storage at 4°C.

### Injectability of nanoparticle containing formulations at room temperature

Pharmaceutical formulations according to the invention have to be liquid or slightly viscous at room temperature to allow injection by means of suitable injection devices attached to a laparoscope. To test the pharmaceutical formulations for this property, the force necessary to fill the syringe through a cannula was assessed. All selected pharmaceutical formulations could by aspirated through the 0.9x40 mm needle into the syringe.

### Dissolution of gels - gel strength

Selected pharmaceutical formulations were investigated for their gel strength as described above. Dissolution of gels in water over time was taken as an indicator for stability of gels after injection into tissue.

The results indicated that 15% of Poloxamer 407 alone could not provide suitable gel stability for the intended application because it loses its gel properties within minutes after overlay with water.

Pharmaceutical formulation containing 15% Poloxamer 407 and 20% Poloxamer 188 started dissolving after 30 minutes of incubation.

Quantum dot loaded formulations composed of 18% to 20% of Poloxamer 407 and 5% to 15% of Poloxamer 188 were the most stable under the experimental conditions described: phase separation between gel and water phase was clearly visible over hours.

### pH values of gels

pH values were measured in water diluted test samples. pH values of "empty" gels range between 6.84 and 7.58 depending on the composition of the samples.

As expected addition of Qdot^{®}655 (in its original borate buffer) led to shift of pH values to 7.03 to 8.38. This is due to the high pH of borate buffer (50 mM pH 8.3)

### DISCUSSION AND CONCLUSION

The aim of this study was to prepare pharmaceutical formulations containing fluorescent nanoparticles having thermo-responsive gelation behavior to be used effectively as injectable formulation for sustained marking of selected tissue areas. Poloxamer based hydrogels were shown to meet these requirements. Poloxamer 407 and 188 have been reported to be the least toxic of commercially available copolymers and is approved for parenteral application.

Viscosity and injectability of 15% Poloxamer 407 formulations were shown to fulfill the necessary requirements. However, Poloxamer 407as single excipient rapidly mixes with water and gel erosion is observed; this would lead to rapid disappearance of fluorescence signal after local injection into tissue. Stability of gels was improved by addition of Poloxamer 188.

Pharmaceutical formulations composed of 18 - 20% Poloxamer 407 / 5% to 10% Poloxamer 188 / 70% or 65% of water or aqueous buffer are slightly viscous at refrigerated temperatures, viscous at room temperature and semisolid at body temperature. Aspiration into syringes and injection of the pharmaceutical formulation into tissue was easy to be performed.

D,L-α-Tocopherol was added to the optimized pharmaceutical formulation since in experience of the inventors formulations with this excipient are stabilized especially when diluted. D,L-α-Tocopherol changes the color of the Qdot^{®}655 preparations and slightly increases viscosity at all temperatures. The fluorescence properties are reduced as compared to identical pharmaceutical formulations without D,L-α-Tocopherol.

### Example 8: Permeability of CACO-2 cells for quantum dots in an inventive

### formulation

The aim of the study was to investigate the permeability of four different pharmaceutical formulations according to the invention across Caco-2 cell monolayer assay. Membrane transport was measured in the apical-to-basolateral (A2B) direction.

### Tested formulations:

- Control formulation (BB): 300µg QDot®655 solution dissolved in 4651 µL borate buffer,
   (50mM pH 8.3).
- Formulation 1: 300µg QDot^{®}655 plus 4651 µL consisting of
   15 % poloxamer 407 /15 % poloxamer 188 / 2% DL-α-Tocopherol / 61% Aqua ad injectabilia.
- Formulation 2: 300µg QDot^{®}655 plus 4651 µL consisting of
   18 poloxamer 407 / 5% poloxamer 188 / 70% Aqua ad injectabilia.
- Formulation 3: 300µg QDot®655 plus 4651 µL consisting of
   18% poloxamer 407 / 10% poloxamer 188 / 65% Aqua ad injectabilia.

### Cell preparation

One vial of Caco-2 cells (ECACC, 09042001), stored in liquid nitrogen, was thawed and seeded in Caco-2 cell media (500 mL DMEM + 10% FBS + 1% L-Alanyl-L-Glutamine + 1% antibiotic/antimycotic + 1% MEM NEAA) in a 75 cm² flask. Cells were passaged two times per week. In order to create a cell monolayer, 2x10⁵ Caco-2 cells (passage 22) were seeded on top of every 24-well plate insert (Millicell 24-well plates, Millipore) in 0.4 mL of Caco-2 media and 26 mL media was added to the bottom compartment. Cells were incubated for 21 days in a CO² incubator with replacement of media every three days.

### Dispersion of quantum dot formulations on Caco-2 cell monolayer

Dispersion of QDot^{®}655 formulations in contact with the Caco-2 cells and medium in the well was investigated using photography under UV light. One 24-well plate without cells was used to optimize camera settings (Camera Canon EOS 350D, lens Tamron 28-75 f2.8, excitation UV lamp 365 nm). 20 µL of each pharmaceutical formulation, or control beads (Fluoresbrite Plain YG 60 micron Microspheres, Polysciences), was added with or without cell culture medium and camera settings were adjusted accordingly. Prior to start of the experiment, the medium was replaced by fresh medium and cells were left to equilibrate for one hour at 37 °C, 5% CO2 (incubator). Different volumes (10, 20 and 40 µL) of each QDot^{®}655 formulation were added to the apical side of the Caco-2 monolayer in cell medium. In parallel, 20 µL of each pharmaceutical formulation was added to cells without medium to enhance the signal. The plate was captured by photography at three time points: 0, 30 and 60 minutes.

### Permeability of quantum dot formulations through the Caco-2 monolayer

Prior to start of the experiment, monolayer integrity was inspected by transepithelial resistance (TEER) measurement, using Millicell ERS Volt-Ohm Meter, according to the manufacturer's instructions. Cells were used for further permeability testing only if the Caco-2 cell monolayer showed good consistency (TEER ≥ 1500 Ohm/cm²).

20 µL of each QDot^{®}655 formulation were added to the apical side of the Caco-2 cell monolayer (in 0.4 mL of medium) in triplicates. Two cell plates were used, one of which was incubated for 1 h and the other for 3 h. Following addition of QDot^{®}655, the plates were incubated on a Vibrating Titramax 1000 Shaker at 37°C. In parallel, 20 µL of QDot^{®}655 were mixed with 400 µL of medium (final concentration 15 ng/µL) and incubated together with cell plates to be used for standard curve preparation. Following 1 h and 3 h of incubation, medium from the apical and basal side (separately) of the cell monolayer from each plate was transferred into two aliquots - one for fluorescence measurement and one for Cd determination (100 µL each). Fluorescence was measured immediately as described below, and samples for Cd determination were kept frozen at - 20°C until analysis.

At both time points, for the purpose of determination of cell-associated concentrations, cells were washed two times with sterile PBS and lysed with 200 µL 0,5% Triton X-100, resuspended by pipette and diluted with an additional 200 µL 0,5% Triton X-100. The lysate was divided into two separate aliquots for fluorescence measurement and Cd determination (100 µL each).

### Fluorescence measurement

The standard curve for each pharmaceutical formulation was generated from fivefold dilution steps, five points (1x, 5x, 25x, 125x, 625x), starting from 15 ng/µL. Pure medium was used as blank.

For each sample, 100 µL of medium from the apical and basal side, as well as cell lysate, was added into a well of a black 96-well plate, and fluorescence was measured at 340nm (exc) and 665nm (ems) on an EnVision 2104 (Perkin Elmer) reader.

### Determination of cadmium concentration

The determination of Cd was performed at the Institute for Medical Research and Occupational Health, Analytical Toxicology and Mineral and Analytical and Toxicology and Mineral and Metabolism Unit (under the supervision of Dr. Sc. Jasna Jurasović, Head of the Analytical Toxicology and Mineral Metabolism Unit).

### Instruments and reagents

Sample decomposition was performed using an UltraCLAVE IV microwave digestion system (Milestone S.r.l., Sorisole, Italy) with integrated software and the cryoLAB cooling system, equipped with 40 quartz vessels (12-mL capacity) and PTFE covers. Cd was determined using an Agilent 7500cx ICP-MS (Agilent Technologies, Waldbronn,Germany) equipped with an integrated autosampler, a Peltier cooled (at 2°C) Scott-type quartz spray chamber, a MicroMist nebuliser, a collision cell, and Ni cones. The instrument is controlled and data collected using MassHunter Workstation software, version B.01.01 (Agilent Technologies, 2010). The laboratory facilities, designed for routine measurement of trace elements, operate under positive pressure maintained by a HVAC (heating, ventilating, and air conditioning) system combined with HEPA filters.

Ultrapure water (18 MΩ cm), obtained with a GenPure system (TKA Wasseraufbereitungssysteme GmbH, Niederelbert, Germany), was used for dilution of all solutions. All standard solutions were prepared from a stock standard solution obtained from PlasmaCAL (SCP Science, Quebec, Canada). Analytical grade nitric acid (65%, Merck, Darmstadt, Germany) was used after purification by sub-boiling distillation in SubPUR sub-boiling system (Milestone S.r.l., Sorisole, Italy). All quartz vessels and polypropylene containers were cleaned with detergent solution, soaked in 10 % HNO₃ for 24 h, and rinsed with ultrapure water three to five times.

### Acid digestion of the samples

0.050 mL of each biological sample was mixed with 0.200 mL of ultrapure 65% nitric acid and 1 mL ultrapure water added in quartz vessel. Microwave digestion was performed in UltraCLAVE IV according to the described procedure (Analytical Protocol AP604-UltraCLAVE, Chapters 4.4-4.9). Due to small amount of sample and reacting acid, the microwave digestion program was modified as shown in Table 1. Following digestion, samples were diluted with ultrapure water to a total volume of 5 mL (i.e. 100 fold dilutions).

**Table 1. Temperature, time, microwave power and pressure conditions for digestion of the cell supernatants/lysates in the UltraCLAVE high-pressure microwave svstem.**

| | T(min:s) | E(W) | T1(°C) | T2(°C) | P(bar) |
|---|---|---|---|---|---|
| 1 | 3:30 | 700 | 70 | 70 | 100 |
| 2 | 15:00 | 1000 | 180 | 70 | 100 |
| 3 | 10:00 | 1000 | 250 | 70 | 140 |
| 4 | 30:00 | 1000 | 250 | 70 | 140 |
| cooling | 40:00 | 0 | 30 | 70 | 20 |

### Determination of Cd by ICP-MS

Prior to measurements, 2 mL of the acid digested and diluted sample solutions were pipetted into PP vials (V=5mL; Sarstedt, Germany) and 50µL of the internal standard solution (p=80 µg/L) were added (the final concentration of the internal standards, Ge, Rh, Lu and Tb, in the samples introduced to ICP-MS was 2 µg/L). Prior to measurements (=60 min), Agilent 7500cx instrument was adjusted to working conditions. Measurements were performed using the instrument's Octopole Reaction System (ORS) and He collision mode ("He-mode"). Sensitivity and stability of the system was checked using the multi-element tune solution (Li, Mg, Co, Y, Tl and Ce in 1% HNO₃, ρ=1µg/L) and formation of oxides (¹⁴⁰Ce¹⁶O⁺/¹⁴⁰Ce⁺=1.2%) and double charged ions (¹⁴⁰Ce²⁺/¹⁴⁰Ce⁺=1.26%) was controlled. Final ICP-MS operating conditions are given in the Table 2. ¹¹¹Cd isotope was measured and ¹⁰³Rh was finally chosen to correct for instrumental drift and matrix effects. Cadmium concentration measurements were also performed in samples treated with medium only (without QDot^{®}655 formulations) for background subtraction purposes.

**Table 2. Agilent 7500cx operating conditions and data acquisition parameters.**

| **Parameter** | **Setting** |
|---|---|
| RF Power (W) | 1550 |
| Outer gas flow (L/min) | 15 |
| Sample Depth (mm) | 8.0 |
| Torch-H (mm) | 0.2 |
| Torch-V (mm) | -0.3 |
| Makeup gas flow (L/min) | 0.18 |
| Carrier gas flow (L/min) 0.90 | 0.90 |
| Nebulizer pump (rps) | 0.08 |
| Helium collision gas flow (mL/min) | 3.4 |
| Extract lens 1 voltage (V) | 0.5 |
| Extract lens 2 voltage (V) | -131 |

### Data analysis

For standard curves, blank values were subtracted from raw data and standard curves were drawn in Microsoft Office Excel 2007 as XY Scatter charts. Linear trendline was applied to the curves, crossing the zero point.

In fluorescence measurements, blank values were subtracted and concentrations (ng/µL) of QDots in samples were calculated from standard curve equation. The results were shown as mean values ± SD.

### RESULTS

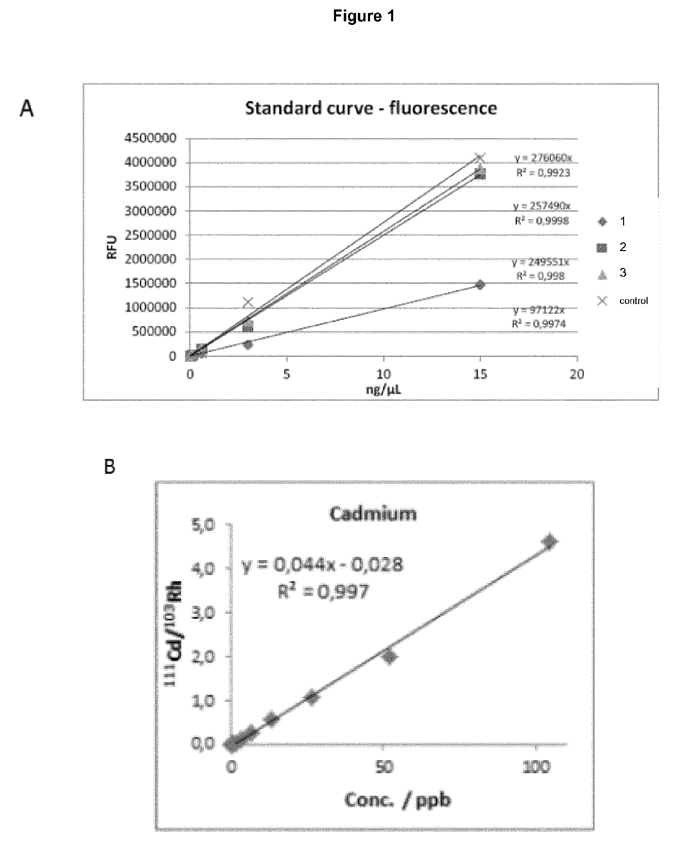

Dispersion of QDot^{®}655 formulations on Caco-2 cell monolayers determined by photography under UV light is shown in Figure 1. A dose dependent increase in fluorescence was seen on the apical side, while the lower compartment did not show any fluroscence, meaning that no QDot^{®}655 were passing the cell layer. Furthermore, it was evident that pharmaceutical formulation 1 showed a significantly lower fluorescence signal as compared to QDot^{®}655 in borate buffer or formulations without D,L tocopherol. The standard curve for the fluorescence measurement showed a linearity between 15 ng/µL and 0.024 ng/µL allowing for comparison of the quantity of QDots (Figure 1A). There was only a minimal difference in the fluorescence intensity between QDot^{®}655 in borate buffer and pharmaceutical formulations 2 and 3, while the pharmaceutical formulation 1 gave a significantly lower signal as already seen in Figure 1. No quantifiable levels of QDot^{®}655 at the basal sides of Caco-2 cells were detected following 1 h or 3h incubation. Therefore, it can be concluded that all tested pharmaceutical formulations have low permeability.

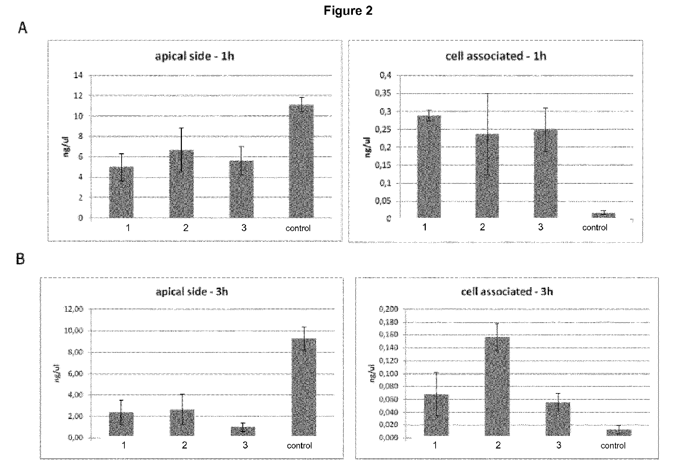

Concentrations of quantum dot formulations at the apical side, as well as cell-associated concentrations determined by fluorimetric measurements, are shown in Figure 2. At 1h, concentrations at the apical side did not differ significantly between pharmaceutical formulations, although formulation 1 produced the weakest signal. Cell-associated concentration at the same time point was significantly lower for the sample in borate buffer compared to the three poloxamer containing formulations.

At 3h, the remaining fluorescence at the apical side was the greatest with the borate buffer containing sample, whereas all poloxamer containing formulations had significantly weaker signals. Cell-associated concentration of pharmaceutical formulation 2 at 3h was increased compared to 1h, and higher than for other pharmaceutical formulations at 3h, indicating most probably greater/more powerful attachment to the cells. Although, 15 ng/µL of each QDot^{®}655 formulation was added to Caco-2 cells, the sums of QDot^{®}655 concentrations at the apical side and cellular level for pharmaceutical formulations 1, 2

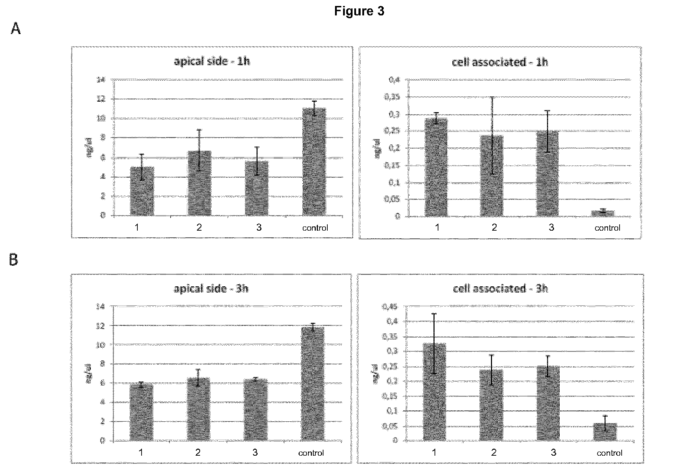

and 3 (Figure 2) were below 15 ng/µL, suggesting that some amount of QDot^{®}655 was washed-out during washing steps. This also indicates that the pharmaceutical formulations did not accumulate inside the cells, but were rather associated with cellular membranes from the outside. The fluorescence signal was clearly associated with the cell layer in the poloxamer containing formulations, while QDot^{®}655 in borate buffer was dispersed in the medium and only slightly attached to the cells (Figure 2, graphs on the right). The corresponding Cd concentrations in the samples are shown in Figure 3. The conclusions regarding permeability and cellular association for the above mentioned inventive formulations correspond to the conclusions made on basis of fluorimetric measurements. At 3h a decrease in fluorescence for poloxamer containing formulations was seen, same as for the Cd level, suggesting a potential instability and degradation of these pharmaceutical formulations due to longer contact with the cells.

### CONCLUSIONS

Since there were no quantifiable levels of QDot^{®}655 at the basal side of Caco-2 cells measured in the A2B direction and determined by both methods (fluorimetric measurement and determination of Cd concentration), it can be concluded that all the quantum dot containing formulations have low permeability. In addition, pharmaceutical formulations 1, 2 and 3 tended to be cell associated since, following washing steps, quantifiable levels of QDot^{®}655 and Cd were measured in those cell lysates, compared to borate buffer.

No significant difference following 1h and 3h incubation was observed with respect to permeability and cellular association. However, lower fluorescence intensity, with the same Cd concentration, was observed for pharmaceutical formulations 1, 2 and 3 following 3h in comparison to 1h incubation. These data suggest a potential instability of QDot^{®}655 in those pharmaceutical formulations due to longer contact with the cells.

### Example 9: Animal Study to Test Local Retention after Injection of an inventive Formulation

The purpose of this study was to evaluate two pharmaceutical formulations (F1 and F2) according to the invention in comparison to a buffer solution of QDots^{®}655 (F0) with respect to tissue retention time and possible local effects. Fluorescence at the injection site was determined by photo imaging and fluorescence microscopy over time and histopathological evaluation of inflammatory signs at the local injection site was performed. Obtained data demonstrate that, following intradermal administration, both tested pharmaceutical formulations (F1 and F2) are retained at the injection site longer and send brighter fluorescence signals from the surface side of skin in comparison to F0. There was no significant difference in fluorescence signal intensity and duration between F1 and F2. This study has been performed according to Good Scientific Practice.

### MATERIALS AND METHODS

### Mice

Male CD1 mice, Charles River (Italy); four weeks old at arrival (BW 24-26 g). Animal related research was conducted in accordance with regional Animal protection legislation (Croatian Official Gazette, NN 135/2006, NN 37/2013 and NN 55/13) and European directive (2010/63/EU) for the protection of animals used for scientific purposes.

The used animal facility is AAALAC (Association for Assessment and Accreditation of Laboratory Animal Care) accredited. Mice are housed in the following ambient conditions: temperature 22 °C± 2, with relative humidity 55 %±10, about 15-20 air changes per hour and 12 hours artificial lighting and 12 hours darkness per day (7 am lights on - 7 pm lights off). Animals have free access to food SDS VRF1 (P), UK and free access to water, distributed in bottles (TECNIPLAST), and filled with drinking water coming from the municipal water main.

All animals were observed daily for changes in behavior, or any signs of distress or toxicity. All injection sites were inspected visually for signs of local intolerance. On the day indicated, animals were euthanatized by administration of an overdose of anaesthetic.

### Preparation of Formulations

- Nanoparticles:: QDot®655 with Concentration of 4.3 µM = 4.3nmol/ml
- Medium:: 50mM borate buffer, pH 8.3

50µl of injection volume should contain 15µg of QDots. Following formulations have been prepared:
558 mg of borate buffer (50mM, pH 8.3) were added to
   - **Batch 22-70:** 7441 mg of formulation consisting of 18% Lutrol F127 / 5% Lutrol F68 / 70% Aqua ad injectabilia.
   - **Batch 22-71:** 7441 mg of formulation 18% Lutrol F127 / 10% Lutrol F68 / 65% Aqua ad injectabilia.
558 mg of Qdots655 were added to
   - **Batch 22-72:** 7441 mg formulation consisting of 18% Lutrol F127 / 5% Lutrol F68 / 70% Aqua ad injectabilia
   - **Batch 22-73:** 7441 mg of formulation 18% Lutrol F127 / 10% Lutrol F68 / 65% Aqua ad injectabilia.
   - **Batch 22-74:** 7441 mg of borate buffer as unformulated control solution

7ml of each formulation are provided for injection experiments. 1ml was stored as back-up formulation. Storage of formulated and control preparations take place at 2°C to 8°C and under light protected conditions. All formulations are liquid at 2°C to 8°C, viscous at ambient temperatures and semi-solid at body temperature, respectively.

### Used Formulations

- F0 = buffer control; quantum dots dissolved in 50 mM borate buffer, 15 µg/50µL; batch 22-74
- F1 = Lutrol formulation with QDots15 µg/50µL; batch 22-72;
- Control 1= vehicle control for F1; Lutrol formulation without Dots; batch 22-70;
- F2= Lutrol formulation with Qdots15 µg/50µL, batch 22-73;
- Control 2 =vehicle control for F2; Lutrol formulation without Qdots; batch 22-71;

### Anesthetics

Ketamine hydrochloride (Narketan, Vetoquinol, Bern, Switzerland). Xylazinehydrochloride (Rompun, Bayer, Germany).

### Injection device

1ml syringes; BD Plastipak; ref 300013, LOT1211009 (exp 10/2017); Sterile, single use needles; BD MICROLANCE Kanuele, 27 G 3/4 0,4x19 mm and 25 G, Becton Dickinson GmbH, PZN: 3086999.

### UV lamp and microscopes

Two identical lamps were used; Vilber Lourmat VL-6.LC at 365 nm.
Light microscope Imager1 with AxioCam ICc1 camera (Zeiss).
Confocal microscope (LSM 510 META, Zeiss). Images were acquired using a 458 nm laser for excitation and 585 nm long-pass filter for detection.
Photo camera: Canon, model EOS 350D.
Photo lens: Tamron, EF 28-75 f2.8.
Software for digital photo images editing: Adobe Lightrom v5.

### Experimental procedures

Intradermal injection:
Mice were anesthetized in order to perform intradermal injections. The dorsolateral skin of the animal was carefully shaved with electric clippers to remove the hair.
The sites were swabbed with 70% ethanol. The needle was inserted into the skin,
bevel up, and held nearly parallel to the plane of the skin without aspiration. A properly performed intradermal injection resulted in a small, round skin welt. Each animal received three intradermal injections of pharmaceutical formulations (formulation, control and F0) on the shaved back. The injection volume of each formulation was 50 µL.

Subcutaneous injection:
The dorsolateral skin on the back of the animal was carefully shaved with electric clippers to remove the hair and cleaned with 70% ethanol. The needle was inserted parallel to the skin of the animal, aspirated to ensure proper placement, followed by injection of the pharmaceutical formulations.
Each animal received three subcutaneous injections of formulations (formulation, control and F0). The injection volume of each pharmaceutical formulation was 50 µL.

### Sampling/photos

At indicated days after application, animals (five per group) were anesthetized i.p. with a combination of ketamine hydrochloride and xylazinehydrochloride. Blood was collected by puncturing the v. jugularis and a. carotis communis into BD tubes with serum separator. Following blood collection, skin from the back area was exposed to UV light and photos taken using a digital SLR camera. Magnification was 0.5x, calculated according to the formula M = f / (g - f); M = magnification, f = the focal length of the lens, g = object distance (between object and lens). For assessment of intradermal application photos were taken both from the surface side and from the subcutaneous side. For assessment of subcutaneous application skin was exposed to UV light from the subcutaneous side and only photos of the subcutaneous side were taken.

Two cryosections were prepared from each skin sample harboring the injection site; skin samples were embedded on the cut edge so that epidermis, dermis and subcutis were revealed. A section encompassing the entire skin thickness was mounted onto a slide and acetone fixed. One of the cryosections was immediately analyzed by confocal microscopy for the presence of fluorescence signal. The other section was stained with hemalaun-eosin and analyzed by light microscopy for signs of inflammation at the injection site. The remaining tissue was placed into 10% formalin.

### Data analysis and statistical evaluation

### Histological analysis was performed as follows:

Digital pictures were taken from each skin sample analysed by confocal microscopy. Pictures were taken at 100x magnification. The intensity of the fluorescence signal was described using a semi-quantitative descriptive method: no fluorescence (0), weak fluorescence (1), moderate fluorescence (2), and bright fluorescence (3).

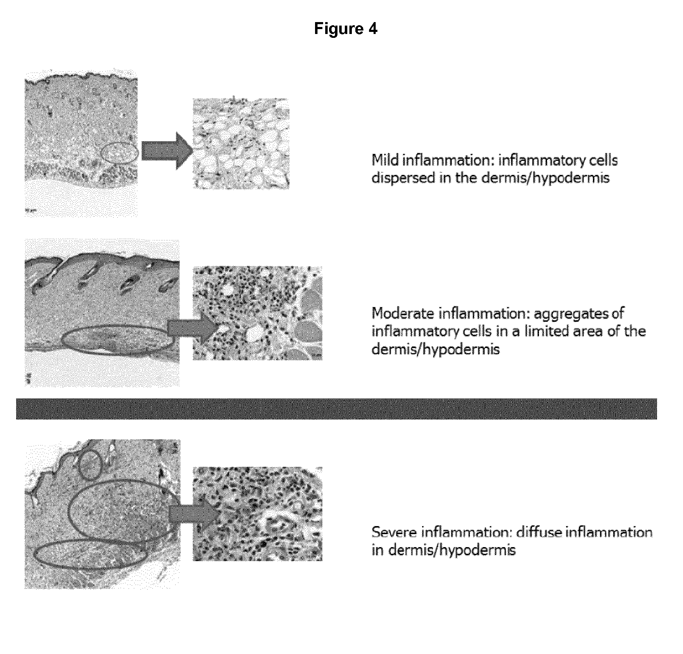

Inflammation was defined as an accumulation of neutrophils and mononuclears in the dermis and/or hypodermis. The site of inflammation was described as "inflammation in dermis" and "inflammation in hypodermis". The intensity of the inflammatory reaction was graded as none (0), mild (1), moderate (2) and severe (3) (Fig. 4). Dispersed inflammatory cells in the dermis/hypodermis were regarded as "mild inflammation". Formation of aggregates of inflammatory cells in a limited area of the dermis/hypodermis was regarded as "moderate inflammation". Presence of diffuse neutrophils and mononuclears in the dermis/hypodermis was regarded as "severe inflammation". Intermediate scores (0/5, 1/2 and 2/3) were used in some cases. The median for each group was calculated.

### RESULTS

### Intradermal injection

### Fluorescence of QDots^{®}655

The fluorescence signal was evaluated from the cutaneous and the subcutaneous side according to the semi-quantitative descriptive scoring system (0 = no fluorescence; 1 = weak fluorescence; 2 = moderate fluorescence; 3 = bright fluorescence) over a period of five days (cutaneous side) and seven days (subcutaneous side) following intradermal application. Two observers in parallel have scored the fluorescence signal. Table 3 summarizes the fluorescence intensity evaluated from the cutaneous side while table 4 summarizes the corresponding scores from the subcutaneous side.

As seen from Table 3, when evaluated from the cutaneous side, pharmaceutical formulations F1 and F2 have shown a prolonged signal, while fluorescence of QDots®655 dissolved in buffer (F0) faded within one day. Contrary to that, as seen from Table 4, subcutaneous evaluation indicated a prolonged signal of QDots^{®}655 dissolved in buffer (F0) when compared to pharmaceutical formulations F1 and F2. These data indicate that QDots^{®}655 dissolved in buffer did not remain at the site of the injection, but have penetrated the subcutaneous space following intradermal injection, while pharmaceutical formulations F1 and F2 maintained the fluorescence signal at the local injection site.

**Table 3: Evaluation of fluorescence from cutaneous side following intradermal injection of QDots^{®}655.**

| Scoring scheme: 0 = no fluorescence; 1 = weak fluorescence; 2 = moderate fluorescence; 3 = bright fluorescence. | | | | | | | |
|---|---|---|---|---|---|---|---|
| Time post injection | score | **Formulations**/ **number of animals** | | | | | |
| | | F0 | F1 | CTRL1 | F0 | F2 | CTRL2 |
| 24h | 0 | | | 5 | | | 5 |
| | 1 | | | | | | |
| | 2 | | 1 | | | | |
| | 3 | 5 | 4 | | 5 | 5 | |
| Comments | | - | - | - | - | - | - |
| 72h | 0 | 2 | 1 | 5 | | 1 | 5 |
| | 1 | | | | 3 | 1 | |
| | 2 | 3 | 1 | | 2 | 2 | |
| | 3 | | 3 | | | 1 | |
| Brownish tissue coloration at administration site | | - | 1/5 | 1/5 | - | 1/5 | - |
| Day 5 | 0 | 4 | 4 | 5 | 3 | 3 | 5 |
| | 1 | 1 | 1 | | 2 | | |
| | 2 | | | | | 1 | |
| | 3 | | | | | 1 | |
| Brownish tissue coloration at administration site | | 2/5 | 2/5 | - | 3/5 | 2/5 | - |

**Table 4: Evaluation of fluorescence from subcutaneous side following intradermal injection of QDots^{®}655.**

| Scoring scheme: 0 = no fluorescence; 1 = weak fluorescence; 2 = moderate fluorescence; 3 = bright fluorescence. | | | | | | | |
|---|---|---|---|---|---|---|---|
| Time post injection | score | **Formulations/ number of animals** | | | | | |
| | | F0 | F1 | CTRL1 | F0 | F2 | CTRL2 |
| 24h | 0 | | 1 | 5 | | 3 | 5 |
| | 1 | | 4 | 4 | 1 | 2 | |
| | 2 | 3 | | | 1 | | |
| | 3 | 2 | | | 3 | | |
| Comments | | - | - | - | - | - | - |
| 72h | 0 | 1 | | 5 | 2 | 5 | 5 |
| | 1 | | | | 3 | | |
| | 2 | 1 | | | | | |
| | 3 | 3 | | | | | |
| Brownish tissue coloration at administration site | | - | 5/5 | - | - | 3/5 | - |
| Day 5 | 0 | 5 | 5 | 5 | 4 | 5 | 5 |
| | 1 | | | | 1 | | |
| | 2 | | | | | | |
| | 3 | | | | | | |
| Brownish tissue coloration at administration site | | 4/5 | 4/5 | - | 4/5 | 4/5 | - |
| Day 7 | 0 | | 5 | 5 | 3 | 4 | 5 |
| | 1 | 5 | | | 2 | 1 | |
| | 2 | | | | | | |
| | 3 | | | | | | |
| Comments | | - | - | - | - | - | - |

### Confocal microscopy

In order to further quantify the fluorescence signal and evaluate the tissue distribution of QDots^{®}655, tissue samples of intradermal injection sites were analysed by confocal microscopy.

Table 5 and Table 6 summarize the results of fluorescence signal evaluation based on photographs taken with confocal microscope. High variation of fluorescence signal was obtained. While a bright signal was seen in all groups 24h following injection, variable fading of the fluorescence intensity was seen in the pharmaceutical formulations F1 and F2 (at 72h, 5 days and 7 days), as well as within the buffer formulation F0 (at 72h, 5 days and 7 days). Both vehicle controls (CTRL1 and CTRL2) did not show a fluorescence signal in any of the groups (data not shown).

**Table 5: Fluorescence intensity as determined by confocal microscopy following intradermal iniection.**

| Time post injection | F0 and F1 | F0 and F2 |
|---|---|---|
| 24h | Bright signal for F0 and F1 | Bright signal for F0 and F2 |
| 72h | Moderate to bright signal for F0 Weak to moderate signal for F1 | Moderate to bright signal for F0 Weak signal with F2 |
| Day 5 | Moderate to bright signal for F0 Weak to moderate signal with F1 | Moderate to bright signal for F0 Variable signal for F2 (without signal or weak; one animal with bright signal) |
| Day 7 | Moderate to bright signal for F0; animals without signal (1/5) Weak signal or without signal (4/5) with F1 | Variable signal for F0; animals without signal (3/5) Weak signal or without signal (3/5) with F2 |

**Table 6: Fluorescence intensity following intradermal injection, median values, n=5.**

| Time post injection | F0 (median) | F1 (median) | F2 (median) | F3 (median) |
|---|---|---|---|---|
| Day 1 | 3 | 3 | 3 | 3 |
| Day 3 | 2 | 1.5 | 2 | 1 |
| Day 5 | 2 | 1 | 2 | 1 |
| Day 7 | 3 | 0 | 0.5 | 0 |

### Evaluation of inflammation

Acute inflammation, defined by the presence of neutrophils among other cell types, was found in all skin samples containing QDots®655. Intradermal application of F0 and both F1 and F2 formulations, induced a mild acute inflammatory reaction at 24 h, characterized by the presence of single inflammatory cells and small accumulations of inflammatory cells in the dermis. Inflammation spread throughout the dermis and into the hypodermis during the observation period. Macrophages, some of which were pigmented, could be identified among inflammatory cells, at 72 h post F1/F2 application until the end of the observation period. The intensity of inflammation varied among animals in the group. Generally, F2 induced a milder inflammation in comparison to F0 and F1. The control induced a mild inflammation that resulted in scar formation along the needle track at the end of the observation period. Table 7 summarizes inflammatory scores following intradermal injection of QDots^{®}655 formulations F1, F2, as well as buffer formulation (F0).

**Table 7: Inflammation intensity following intradermal injection, median values, n=5.**

| Time post injection | F0 (median) | Controls (median) | F1 (median) | F2 (median) |
|---|---|---|---|---|
| 24h | 1 | 1 | 1 | 1 |
| 72h | 2 | 1 | 2 | 1.5 |
| Day 5 | 2 | 1 | 2.5 | 1 |
| Day 7 | 2 | 1 | 1 | 1 |

### Subcutaneous injection

### Fluorescence of QDots^{®}655

The fluorescence signal was evaluated from the subcutaneous side according to the semiquantitative descriptive scoring system (0 = no fluorescence; 1 = weak fluorescence; 2 = moderate fluorescence; 3 = bright fluorescence) over a period of seven days following subcutaneous application. No signal was detectable from the cutaneous side. Brownish coloration of the tissue was observed in two animals from subcutaneous side seven days following application of F2. Table 8 summarizes the fluorescence intensity evaluated from the subcutaneous side. Pharmaceutical formulations F1 and F2 gave a slightly weaker signal when compared to the buffer formulation (F0). Individual photographs show that the buffer formulation gave a more dispersed signal, while F1 and F2 formulations appear to be more localized around the injection site.

**Table 8: Evaluation of fluorescence from subcutaneous side following subcutaneous injection of QDots®655. Scoring scheme: 0 = no fluorescence; 1 = weak fluorescence: 2 = moderate fluorescence: 3 = bright fluorescence.**

| Time post injection | score | **Formulations/ number of animals** | | | | | |
|---|---|---|---|---|---|---|---|
| | | F0 | F1 | CTRL1 | F0 | F2 | CTRL2 |
| 24h | 0 | | | 5 | | | 5 |
| | 1 | | 1 | | | 1 | |
| | 2 | 2 | 4 | | 1 | 2 | |
| | 3 | 3 | | | 4 | 2 | |
| Comments | | - | - | - | - | - | - |
| Day 3 | 0 | | 2 | 5 | | 4 | 4 |
| | 1 | 1 | 3 | | 2 | | |
| | 2 | 4 | | | 2 | | |
| | 3 | | | | | | |
| Comments | | - | - | - | - | - | - |
| Day 7 | 0 | 5 | 3 | 5 | | 3 | 5 |
| | 1 | | 2 | | 3 | 2 | |
| | 2 | | | | 2 | | |
| | 3 | | | | | | |
| Brownish tissue coloration at administration site | | - | - | - | - | 2/5 | - |

### Confocal microscopy

The analysis of samples by confocal microscopy confirmed the macroscopic findings. While at 24h following injection, a bright signal could be seen in all three pharmaceutical formulations (F0, F1, F2), it was observed that the signal faded away over the period of seven days. Both vehicle controls (CTRL1 and CTRL2) did not show a fluorescence signal in any of the groups.

**Table 9: Fluorescence intensity determined by confocal microscopy following subcutaneous injection.**

| Time post injection | F0 and F1 | F0 and F2 |
|---|---|---|
| 24h | Bright signal for F0 and F1, some animals with weaker signal | Bright signal for F0 and F2 |
| 72h | Moderate signal for F0 Fading of signal with F1 and some animals without signal | Moderate to bright signal for F0 Fading of signal with F2 and some animals without signal * |
| Day 7 | No signal for F0 and F1 | Weak or moderate signal for F0; some animals without signal Weak signal for F2 and some animals without signal |

| | | |
|---|---|---|
| *Group of 4 animals, since one animal from the group was found dead. | | |

**Table 10: Fluorescence intensity following subcutaneous injection, median values, n=5.**

| Time post injection | F0 (median) | F1 (median) | F0 (median) | F2 (median) |
|---|---|---|---|---|
| Day 1 | 3 | 1 | 2 | 3 |
| Day 3 | 2 | 2 | 1.5 | 0* |
| Day 7 | 0 | 0 | 1.5 | 0.5 |

| | | | | |
|---|---|---|---|---|
| * Median is given for the group of 4 animals, since one animal from the group was found dead. | | | | |

### Evaluation of inflammation

Subcutaneous application of F0, and both F1 and F2 formulation, induced an acute inflammatory reaction in the hypodermis already on day one post application. The inflammatory reaction was more pronounced in animals treated with F1 and F2 in comparison to F0. Occasionally, small accumulations of brownish amorphous material were identified in the hypodermis/subcutis. Macrophages, some of which were pigmented, were observed at three days post injection. The intensity of the inflammatory reaction decreased over time in all groups, but neutrophils could be identified throughout the entire duration of the experiment. However, individual variations were observed among animals. The control induced a mild reaction that was resolved by the end of the observation period.

**Table 11: Inflammation intensity following the subcutaneous application, median values, n=5.**

| Time post injection | F0 (median) | F1 (median) | F0 (median) | F2 (median) |
|---|---|---|---|---|
| 24h | 1 | 1 | 2.5 | 2 |
| 72h | 0 | 1 | 0 | 1* |
| Day 7 | 0 | 0 | 1 | 1 |

| | | | | |
|---|---|---|---|---|
| * Median is given for the group of 4 animals. | | | | |

### Clinical observations

Animal appearance, as well as food and water intake, was normal in all study groups. The animals displayed normal species specific behaviour. There were no macroscopic signs of inflammation, or necrosis at injection sites. Brownish tissue coloration at administration site after intradermal injections was observed as small spots from cutaneous side, as stated in Table 2 with results.

Note: *one animal has been found dead and excluded from further evaluation due to cannibalism.

### DISCUSSION AND CONCLUSIONS

Single intradermal or subcutaneous application of QDots^{®}655 dissolved in buffer (F0) and two different QDots^{®}655 formulations marked as F1 and F2 resulted in visible red fluorescence signal after exposure to UV light. Close-up photographs of skin surface (cutaneous) side following intradermal injection, have clearly shown that the signal associated with the F0 formulation was less intensive, or mostly not visible, at 72h hours post application, whereas both pharmaceutical formulations F1 and F2 were still clearly visible at 72h post application in the majority of the animals. Moreover, upon exposure to UV light five days post application, fluorescent spots of the F2 formulation were still visible in two animals. No fluorescent signals were present seven days post application. However, inspection of the subcutaneous side, following intradermal injection, revealed intensive fluorescence signals 24h post application both for F0 and F1/F2 formulations. At 72h the signal on the subcutaneous side was brighter for F0 than for F1 and F2. Fluorescence signals were practically not present at days three to seven post application. Therefore, it can be concluded that QDots^{®}655 dissolved in buffer penetrate rapidly through the dermis into the subcutaneous space, while the pharmaceutical formulations F1/F2 withhold the QDots^{®}655 at the site of the (intradermal) injection. Confocal microscopy confirmed the macroscopic results: at 24h following intradermal injection of F0, a bright florescence signal was observed, that progressively faded away in the course of seven days post application. The F1 formulation gave a bright signal at 24h, which faded away over time, but a weak signal was still present at seven days post application. The F2 formulation also gave a bright signal at 24h post application, but faded away during the observation time. Quantum Dots were distributed non-homogeneously mostly throughout the epidermis when formulated, and no red fluorescence was observed in hair follicles. Quantum Dots were found in the dermis, and during the observation period they were progressively diffusing towards the hypodermis. F0 and F1/F2 induced an acute inflammatory reaction characterized by accumulation of inflammatory cells at 24h post application. Intradermal injection of F0 and F1 caused a brighter inflammation than F2. Injection of pharmaceutical formulations corresponding to F1 and F2, but without QDots^{®}655, induced a mild inflammation resulting in scar formation along the needle track by the end of observation period.

Overall, it can be concluded that pharmaceutical formulations F1/F2 of Quantum Dots maintain a prolonged fluorescence signal at the site of injection following intradermal injection, accompanied by a mild inflammatory reaction, that fades away in the course of the observation period.

Following single subcutaneous injection, upon exposure to UV light, the fluorescence signal could be seen at 24h post application with F0 (buffer) and with both pharmaceutical formulations on closeup photographs. At 72h the fluorescence signal on the subcutaneous side was brighter in the F0 group than in F1/F2 groups. Fluorescence signals were no longer visible at day seven after application. Also, brownish coloration of tissue was observed at some injections sites five days post injection. Confocal microscopy showed that, following subcutaneous injection of F0, a bright fluorescence signal was captured at 24h post application and progressively faded away in the course of seven days. The F1 formulation also gave a bright signal at 24h after application, but faded away over time and completely disappeared at day seven.

Similarly, the F2 formulation gave a bright signal at 24 h, getting weaker at 72h after application, but was still present but very weak seven days post application. Fluorescence signals were diffusely found in the hypodermis/subcutis. Contrary to intradermal injection, higher individual variations were detected among animals from the same group following subcutaneous injection. This is probably due to dispersion of the applied volume, since the subcutaneous space in mice is a large virtual space. As visible in close-up photos, QDots^{®}655 tend to spread throughout the subcutis due to high mobility of skin in mice.

Generally, the fluorescence signal was obviously more dispersed for F0 than for F1/F2. Additionally, mainly at later time points, it was not possible to detect with accuracy the exact injection site in some skin samples, which made the sampling for confocal microscopy more difficult. Nevertheless, the signal intensity recorded at close-up, was mostly in concordance with confocal microscopy. Subcutaneous application of F0 and F1/F2 induced an acute, mild to moderate inflammatory reaction in the deep hypodermis/subcutis already during the first 24 hours post application. Intensity of the inflammatory reaction decreased in all groups following subcutaneous application already at 72 hours after application, although there were individual variations among animals. Control formulations, corresponding to F1 and F2, but without Quantum Dots, also induced a mild inflammation that was resolved by the end of the observation period. It should be taken into account that mild inflammation is always present due to needle insertion into the skin.

### Several conclusions can be made from the study:

1. Following intradermal application, F0 is diffusing to the subcutaneous side much easier than F1/F2. This explains a brighter fluorescence signal when inspected from the subcutaneous side. Close-up photos showed that, following intradermal injection, F1/F2 formulations had a lower penetration rate towards the subcutaneous side in comparison to F0.
2. There was no significant difference in fluorescence signal intensity and duration between F1 and F2 on the cutaneous and subcutaneous side. The mouse skin consists of an external epithelium (epidermis), a thick layer of connective tissue (dermis), and a layer of adipose tissue (hypodermis or panniculus adiposus). A thin layer of striated muscle separates the skin from other structures. The injection is made into the outer layers of the skin and F1/F2 obviously spread more slowly throughout the dermis during the observation period. Also, there is a higher possibility of some leakage after intradermal application of F1/F2 due to the viscosity of the pharmaceutical formulations, which may also produce some variability. Diffusion of F1 and F2 from the epidermis into deeper dermis/hypodermis was probably limited by the basement membrane and dermal connective tissue, so only brownish coloration of the subcutis was visible at later time points, mostly without fluorescent signal from the subcutaneous side. This was confirmed by observations from the surface side, since fluorescence spots of F1 and particularly F2 were intensive for a longer period of time than F0.
3. Successful injections were easily visible from the surface side, and compared to F0, fluorescent spots from F1 and F2 were well defined, better assembled in more limited areas and more intensive for a longer period of time. Obtained data demonstrate that following intradermal injection, F1 and F2 are better retained at the site of injection and produce a brighter fluorescence signal than F0.
4. Following subcutaneous injection of F0 and F1/F2, the non-homogeneously distributed red fluorescence signal was concentrated in the hypodermis and between the underlying muscle fibers. With time, QDots diffused in both directions - towards the dermis and the subcutis. Anatomical conditions edict a more localized signal following intradermal application, when compared to subcutaneous. However, even after subcutaneous application, F1 and F2 related signal was more homogenous than that of F0. In general, the signal from F0 was longer visible after subcutaneous application than after intradermal application but more dispersed.
5. Generally, mild to moderate inflammatory signs were seen in all injections, even in pharmaceutical formulations without QDots®655.Inflammatory reaction was most prominent at 24h and stronger following intradermal vs. subcutaneous injection. F2 formulation induced a milder inflammatory reaction than F1. Inflammatory signs faded away in all pharmaceutical formulations with time.

### Example 10: Evaluation of QDot 655 binding on human colon tissue

Membrane binding of QDot 655 was evaluated in cryo tissue sections of human colon cancer and non-malignant colon epithelium using standard immunohistochemical methods for incubation, washing and detection of QDot 655. For control purposes the membrane marker (CDH17) was used to demonstrate specific membrane binding pattern for the conditions and tissue samples used. Binding of QDot 655 was probed by using three 3.5% QDot 655 preparations, buffer (PBS) and two poloxamer gel formulations, F4 and F5, respectively. Furthermore the membrane binding experiments of CDH17 and QDot 655 were completed by respective negative controls (antibody isotype control, PBS, Gel-F4 and Gel-F5). Binding studies were executed at room temperature and 37°C.

### A) Manufacturing protocol of tested QDot 655 gel formulation F4 and F5 (3.5%)

### 1. List of ingredients and eauipment used:

QDot®655 colloidal solution in 50 mM borate buffer from LTC Life Technologies Corporation, Carlsbad, CA, US C47013

Kolliphor® P407 BTC Europe GmbH, Burgbernheim

Kolliphor® P188 BTC Europe GmbH, Burgbernheim

Aqua ad injectabilia Brau, Melsungen

### Antibodies:

- Human Cadherin-17 MAb (CHD17), mouse monoclonal, clone 141713 (Biomedica (R&D); RD-MAB1919)
- Isotype control, mouse IgG1 (DAKO; X0931)

**Table 12: Composition of final (3.5%) QDot containing formulation F4**

| Excipients and substance | Concentration [%] | net weight in 20 g |
|---|---|---|
| Kolliphor® P407 | 18.00 % w/w | 3.60 g |
| Kolliphor® P188 | 5.00 % w/w | 1.00 g |
| Aqua ad inj. | 73.50 % w/w | 14.70 g |
| purified QDots®655 | 3.50 % w/w | 0.70 g |

**Table 13: Composition of final (3.5%) QDot containing formulation F5**

| Excipients and substance | Concentration [%] | net weight in 20 g |
|---|---|---|
| Kolliphor® P407 | 18.00 % w/w | 3.60 g |
| Kolliphor® P188 | 10.00 % w/w | 2.00 g |
| Aqua ad inj. | 68.50 % w/w | 13.70 g |
| purified QDots®655 | 3.50 % w/w | 0.70 g |

2 Procedure: Purification of QDot®655 original solution by repeated filtration steps 1 g of QDot®655 original solution was transferred into Vivaspin cartridge and 4 g of Aqua ad injectabilia were added. This Vivaspin cartridge was centrifugated at 3000 g for 30 min at 21°C. Subsequently the Filtrate 1 was removed from the lower part of the cartridge.

Then 4 ml of aqua ad injectabilia were added to the QDot®655 concentrate in the upper compartment of the cartridge, carefully resuspended and again centrifuged (3000 g / 30 min / 21°C). Filtrate 2 was removed from the lower part of the cartridge. This procedure was repeated once.

Finally Aqua ad injectabilia was exactly added to the QDot®655 concentrate in the upper compartment of the cartridge (taking the tare weight into consideration) to obtain the initial weight of 1 g. The QDot®655 concentrate was carefully resuspended and stored at 4°C under light protection in glass vials.

### 3. Preparation Procedure

Kolliphor P407 and Kolliphor P188 were accurately weighted into a glass beaker which was placed in a preheated (70°C - 80°C) water bath.

After the Poloxamer was completely melted; the appearance was colorless, transparent, homogeneous, and liquid. Aqua ad injectabilia was added to the molten mass and dispersed by stirring. Immediately after dispersing preparation was stored under refrigerated conditions (2°C-8°C) until appearance is colorless, homogeneous and transparent. Aqua ad injectabilia wass added o obtain final net weight of 50g of the F4 or F5 carrier gel.

19.3 g of the F4 or respectively F5 carrier gel was accurately weight in a glass beaker and 700 mg of purified QDot®655 suspension was added and slowly mixed until the formulation appears homogeneous, orange red and transparent. The product was transferred into glass vials, sealed and stored under protection from light at a cool place.

For subsequent use the QDot 655 dipeptide quantum dots (100%) were diluted with PBS to yield a final working concentration of 3.5%. QDot 655 gel formulation F5 (7%) were diluted with Poloxamer gel formulation F5 to yield a final working concentration of 3.5%. Prepared dilutions were mixed for 5 min by overhead mixing at room temperature, final dilutions were stored at 4° under light protection.

### B. Study design and goal

Aim of this project is the evaluation of the potential degree of membrane binding properties of QDot 655 in an inventive formulation at room temperature (RT, 24°C) as well as at 37°C to colon cancer tissue and to non-malignant colon tissue. Therefore the binding of QDot 655 is evaluated for an aqueous buffered QDot 655 solution (QDot 655-buffer) as well as for inventive QDot 655 preparation (QDot 655-gel F4 and QDot 655-gel F5). The analysis allows the comparison of potential membrane binding properties of QDot 655 under various conditions (i.e. buffer, gel, RT, 37°C) and tissue types (i.e. cancer, non-malignant). This study was planned, performed, monitored, recorded, archived and reported according to the Quality Management System of ORIDIS Biomarkers.

### C. Materials and Methods

### Human cryo tissue samples

Anonymized cryo tissue samples and pathological data of adenocarcinoma of the colon (n=3; table 14) or normal colon epithelia (n=3; resection margins of colon cancer patients) were purchased from the EN/ISO 9001:2008 certified Biobank Graz (Graz, Austria). Tissue histology (table 14) was re-evaluated and confirmed by a trained pathologist. Tumor and non-malignant epithelia tissue area was quantified using Aperio ScanScope software (Leica Biosystems, Germany) in order to quantify the approximate tissue content available for evaluation.

**Table 14: Colon tissue samples and pathological data.**

| Sample ID | Histology | Scan file | Qualified tissue size (mm²) | Age | Gender | Stage | pTNM | Grade |
|---|---|---|---|---|---|---|---|---|
| 40935 | non-malignant epithelia | 2571407_3 | 17.8 | 62 | Male | -- | -- | -- |
| 4792 | non-malignant epithelia | 2571407_5 | 11.3 | 80 | Male | -- | -- | -- |
| 5152 | non-malignant epithelia | 2571407_8 | 13.6 | 83 | Female | -- | -- | -- |
| 7545 | adenocarcinoma | 2571407_2 | 9.08 | 52 | Male | III | pT4N2M1 | G3 |
| 4738 | adenocarcinoma | 2571407_4 | 12.8 | 65 | Female | III | pT4N2Mx | G3 |
| 26 | adenocarcinoma | 2571407_10 | 19.6 | 69 | Male | IV | pT2N2aM1 | G3 |

### QDot 655 and antibody binding

Cryo tissue sections with a thickness of 5 µm have been used for subsequent analysis of QDot 655 binding as well as analysis of CDH17 and the control antibody immune reactivity. Cryo tissue sections were fixed for 10 min in acetone (4°C), incubated with Bovine Serum Albumin (20 min, 2% (w/v) BSA, RT). Next tissue sections were incubated with QDot 655-buffer (3.5%), QDot 655-gel F4/F5 (3.5%) and controls, respectively for one hour at RT or 37°C. After incubation of the tissue slides with QDot 655 preparation and controls, the tissue slides were washed 3 times for 10 min. Finally slides had been DAPI stained and covered.

Alternatively, after were blocking the endogenous peroxidase activity (20 min, 1% H₂O₂, room temperature) and incubation with Bovine Serum Albumin (20 min, 2% (w/v) BSA, RT) the fixed sections were incubated with CDH17 and control antibody dilution (1 µg/ml, 1 h), respectively. Next, slides were washed (3 times, 10 min) and subsequently incubated with ChemMate Envision HRP mouse/rabbit (bottle A, 30 min) and further processed according to the manufactures guideline. Finally sections were covered with Entellan®.

### Sample evaluation and documentation

In the region of interest (adenocarcinoma of the colon or non-malignant colon epithelia), membrane binding of QDot 655-buffer or QDot 655-gel F4/F5 in epithelial cells were evaluated. Digital images of representative areas were captured at a Zeiss Axioplan microscope using an Axiocam MRm camera from Zeiss (Jena, Germany) at a total microscope magnification of 200x.
- Combined images of DAPI (Excitation: 365 nm; Emission: 445/50 nm) and QDot 655 fluorescence (Excitation: 546/12 nm; Emission 590 nm) were recorded at a 200x microscope magnification and subsequently analyzed for percentage of luminal membrane showing QDot 655 fluorescence.
- Lengths of total luminal membrane and luminal membranes with signal were calculated using image analysis software, ImageScope (Aperio, UK).
- Luminal membrane positive for QDot 655 flourescence were expressed as % of total luminal membrane.
- The average membrane binding and standard deviation was calculated for each experimental condition tested (i.e. tissue sample ID, QDot type/control and temperature) based on the three batches analyzed for each experimental condition.
- The average membrane binding and standard deviation was calculate for each group of experiments (i.e. tissue type, QDot type/control and temperature)
- For statistical comparison of membrane binding between tissue types (i.e. non-malignant colon vs cancer epithelium), temperatures and formulations, membrane binding was compared using Mann-Whitney U test.
- Statistical calculations were performed using SigmaPlot® (Systat Software, Germany)

### Results

**Table 15: Summary of luminal reacticity (%) in non-malignant and cancer epithelium atroom temperature and 37°C. Given are averages and standard deviations oft he % of luminal membrane reactivity for threee tissue samples and three independent experiments for each tissue sample for each given condition.**

| Condition | RT/24°C | | 37°C | |
|---|---|---|---|---|
| | non-malignant | tumor | non-malignant | tumor |
| CDH17 | 95 ± 0 | 95 ± 0 | n.d. | n.d. |
| Isotype control | 0 ± 0 | 0 ± 0 | n.d. | n.d. |
| QDot 655-buffer | 0 ± 0 | 28 ± 20 | 0 ± 0 | 16 ± 26 |
| PBS | 0 ± 0 | 0 ± 0 | 0 ± 0 | 0 ± 0 |
| QDot 655-gel F4 | 0 ± 0 | 22 ± 29 | 0 ± 0 | 67 ± 20 |
| Gel-F4 | 0 ± 0 | 0 ± 0 | 0 ± 0 | 0 ± 0 |
| QDot 655-gel F5 | 0 ± 0 | 13 ± 16 | 0 ± 0 | 26 ± 39 |
| Gel-F5 | 0 ± 0 | 0 ± 0 | 0 ± 0 | 0 ± 0 |

- Controls:
   o None of the negative controls (antibody isotype control, PBS, Gel-F4 and Gel-F5) indicated for any detectable membrane signal, excluding also potential autofluorescence.
- CHD17:
   o Specific membrane immune reactivity to CDH17 was observed in cells of nonmalignant epithelia colon tissue and adenocarcinoma of the colon. The observed membrane reactivity was present in at least 95% of the entire membrane. The negative control (antibody isotype) lacked any detectable membrane reactivity.
- QDot 655-buffer solution:
   o No membrane binding of QDot 655 in PBS (QDot 655-buffer) was observed in non-malignant colon tissue after incubation for 1 hour at RT or 37°C
   o A luminal membrane binding of QDot 655-buffer was detected in all three adenocarcinoma samples at RT (QDot 655 binding to 8% - 46% of luminal membrane) and in two of three samples (ID 4738 and ID 26) after incubation at 37°C (QDot 655 binding to 10% - 39% of luminal membrane)
   o Intracellular binding (cytoplasm and nucleus) was observed in non-malignant colon tissue and adenocarcinoma of the colon
- QDot 655-gel formulation F4:
   o No membrane binding of QDot 655 in gel formulation F1 was detected in nonmalignant epithelia colon tissue after incubation for 1 hour at RT or 37°C
   o A luminal membrane binding of QDot 655 in gel formulation F4 was detected in all three adenocarcinoma samples after incubation at RT (QDot 655 binding to 10% - 32% of luminal membrane) or 37°C (QDot 655 binding to 56% - 83% of luminal membrane)
   o Intracellular binding (cytoplasm and nucleus) was observed in non-malignant epithelia colon tissue and adenocarcinoma of the colon
- QDot 655-gel formulation F5:
   o No membrane binding of QDot 655 in gel formulation F5 was detected in nonmalignant colon tissue after incubation for 1 hour at RT or 37°C
   o A luminal membrane binding of QDot 655 in gel formulation F5 was detected in all three adenocarcinoma samples after incubation at RT (QDot 655 binding to 7% - 24%% of luminal membrane) or 37°C (QDot 655 binding to 24% - 28% of luminal membrane)
   o Intracellular binding (cytoplasm and nucleus) was observed in non-malignant colon tissue and adenocarcinoma of the colon

In summary, a membrane binding of QDot 655 using QDot 655-buffer, QDot 655-gel F4 or QDot 655-gel F5 in non-malignant epithelial tissue had never been observed. Membrane binding of QDot 665 using QDot 655-buffer, QDot 655-gel F4 or QDot 655-gel F5 had only been observed in cancer epithelium and was furthermore limited to the luminal membrane site. The observed luminal membrane binding pattern of QDot 655 using QDot 655-buffer, QDot 655-gel F4 or QDot 655-gel F5 is due to QDot 655 interaction with the luminal membrane site, since no such signal pattern had been observed in the respective controls PBS, Gel-F4 and Gel-F5. The difference of luminal QDot 655 binding using QDot 655-buffer, QDot 655-gel F4 or QDot 655-gel F5 between non-malignat and cancer epithelium is statistically significant (p < 0.05).

No significant differences had been observed of QDot 655 luminal membrane reactivity in tumor between QDot 655-buffer, QDot 655-gel F4 or QDot 655-gel F5 at RT and 37°C with the exception for QDot 655-gel F4 at 37°C which exhibited a significantly higher luminal membrane reactivity compared to QDot 655-buffer and QDot 655 gel-F5. However, a statistical significant difference (p < 0.05) at RT and 37°C was observed between the luminal membrane reactivity of CDH17 and QDot 655 using QDot 655-buffer, QDot 655-gel F4 and QDot 655-gel F5, respectively. Furthermore data scattering was apparently lacking for CDH17 membrane and luminal membrane reactivity (below detection sensitivity). The observed reactivity of CDH17 was highly consistent from batch to batch and also between samples as expected for a standardized IHC assay. Using the same method, the opposite was observed for QDot 655 binding using QDot 655-buffer, QDot 655-gel F4 and QDot 655-gel F5, respectively. Here a significant data scattering between batches was observed indicating for the weak and unspecific nature of the QDot 655 interaction observed at the luminal membrane in the cancer epithelium for QDot 655-buffer, QDot 655-gel F4 and QDot 655-gel F5, respectively.

A statistically significant difference of QDot 655 luminal membrane reactivity between RT and 37°C has not been observed for QDot 655-buffer and QDot 655-gel F5 however respectively observed for QDot 655-gel F4 (p < 0.05). This observation might indicate for a formulation specific effect of F4 rather than for QDot 655 dependent effect and this finding is potentially relevant for the use as injectable endoscopy formulation into gut tissue at body temperature. This interpretation is supported by the finding that no such difference was observed for QDot 655 reactivity using a simple PBS diluted QDot 655 preparation (QDot 655-buffer).

The obvious difference in the binding pattern between non-malignant and cancer epithelium is most likely due to the difference in the composition of the cell membrane (i.e. expressed membrane proteins, lipid composition and glycosylation pattern etc.) observed between non-malignant and cancer epithelium. Furthermore the observed binding pattern to the luminal membrane in cancer epithelium is most likely of unspecific nature.

### Example 11: Measurement of viscosity

Viscosity of gel-F4/F5 and the formulations QDot 655-gel F4/F5 has been determined at 24°C, 37°C and 41 °C.

For the determination of the viscosity for each measuring temperature a shear rate ramp was used, whereas the shear rate was continuously increased in a defined time window. With this test the flow behavior of the test items can be characterized.

### Results:

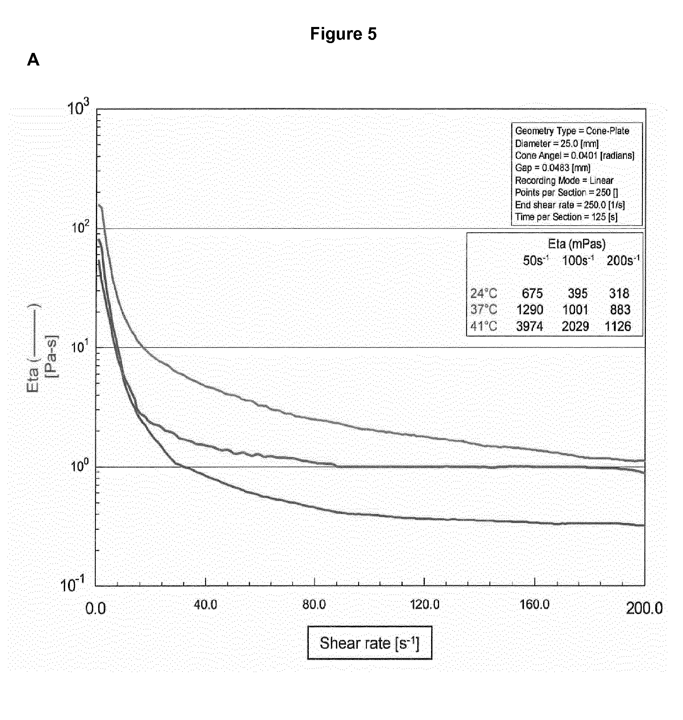

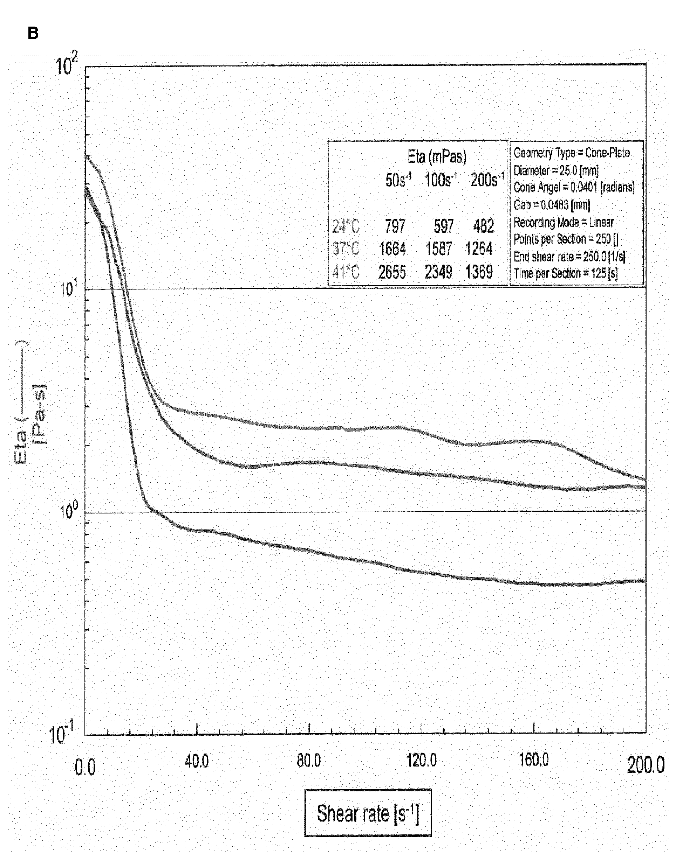

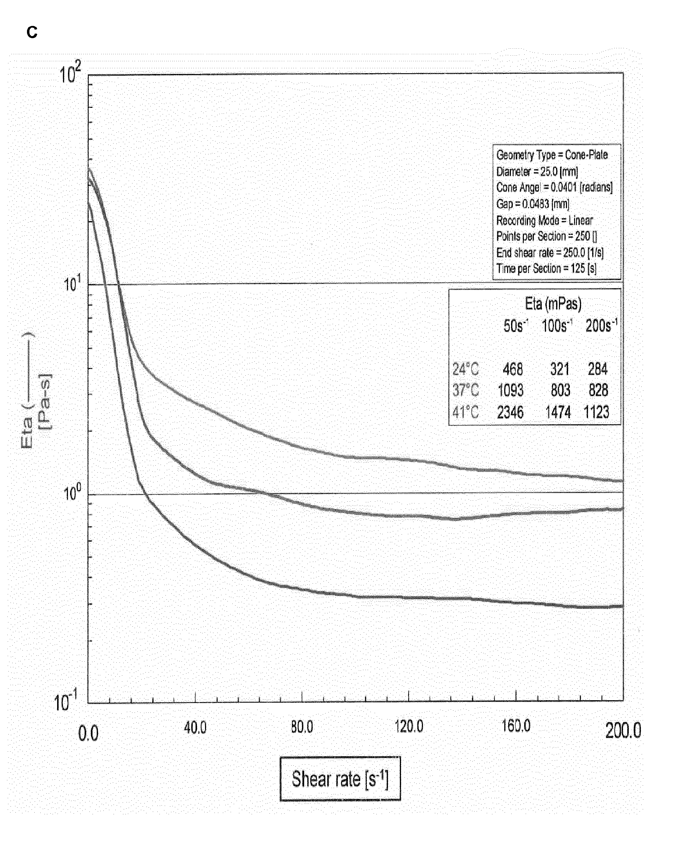

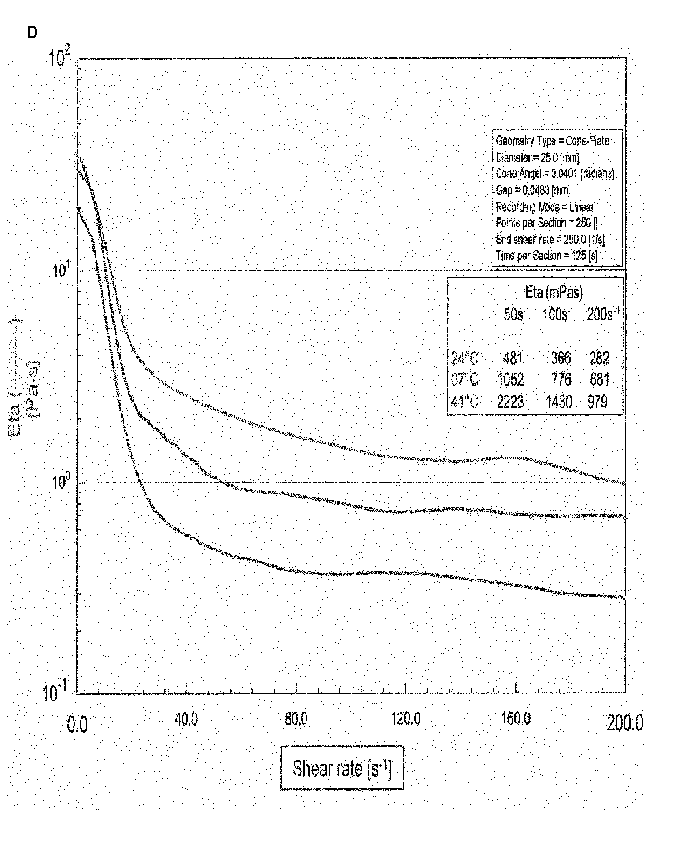

All test items show a structured flow behavior (see Figure 5) that means with increasing shear rates the viscosity decreased. In the shear range of 1 s⁻¹ to ca. 50 s⁻¹ the decrease of the viscosities is extremely strong (from > 10000 mPa to < 4000 mPa). Afterwards the decrease of viscosities up to shear rate of 200 s⁻¹ is only moderate.

The measurements show that there is an increase in the viscosities with increasing temperature from 37°C to 41 °C.

## Claims

1. A pharmaceutical formulation for injection comprising 5% to 50% per weight of a poloxamer, 0.1 nM to 10.0 µM nanoparticles and water or an aqueous buffer, wherein the pharmaceutical formulation is liquid at 4°C - 30°C and forms a gel at 37°C.

2. The pharmaceutical formulation according to claim 1, comprising 17% to 20% per weight of poloxamer 407 and 3% - 15% per weight poloxamer 188.

3. The pharmaceutical formulation according to claim 1 or 2, wherein the nanoparticles are selected from the group consisting of: semiconductor nanoparticles, quantum dots, dye-doped silica particles, dye dope calcium particles, dye labeled liposomes, fluorescent carbon nanoparticles, iron oxide nanoparticles, gold nanoparticles, gold nanorods, gold nanoshells, nanoparticles containing organic chromophores, nanoparticles containing indocyanine green, silver/organic dye composites nanoparticles, nanoparticles containing a gadolinium oxide core, and combinations thereof.

4. The pharmaceutical formulation according to claim 3, wherein the nanoparticles are semiconductor nanoparticles or quantum dots.

5. The pharmaceutical formulation according to claim 4, wherein the quantum dots are cadmium selenide or cadmium telluride quantum dots coated with zinc sulfide.

6. The pharmaceutical formulation according to any one of claims 1 - 5, wherein the aqueous buffer is a phosphate buffer or phosphate buffered saline.

7. The pharmaceutical formulation according to any one of claims 1 - 6, wherein the nanoparticles contain a ligand selected from the group consisting of peptides, antibodies, and oligonucleotides.

8. The pharmaceutical formulation according to any one of claims 1 - 7, wherein the nanoparticles contain a pharmaceutically active agent.

9. The pharmaceutical formulation according to any one of claims 1 - 8 for use as a marker of bodily areas applied by injection directly into an organ or diseased body region.

10. The pharmaceutical formulation according to any one of claims 1 - 9 for use as a marker of tumor areas to enable surgical precision of tumor removal.

11. The pharmaceutical formulation according to any one of claims 1 - 10 for use as a marker of gastrointestinal tumor areas applied by injection during an endoscopic or surgical procedure.

12. The pharmaceutical formulation according to any one of claims 1 - 10 for use as a marker of areas in the skin.

13. The pharmaceutical formulation according to claim 12 for use as a marker of areas in the dermis of the skin.

14. The pharmaceutical formulation according to any one of claims 1 - 3 or 6 - 8, wherein the nanoparticles are super paramagnetic nanoparticles for use in photothermal therapy.

15. The method for preparation of a pharmaceutical formulation according to any one of claims 1 - 14 comprising the following steps:
a) dissolving at least one poloxamer in water or in an aqueous buffer;
b) stirring for at least 20 hours at 4°C; and
c) adding at least one nanoparticle.
